(19) Europäisches Patentamt
European Patent Office
Office européen des brevets

(11) EP 2 949 674 A1

# (12) DEMANDE DE BREVET EUROPEEN

(43) Date de publication:
**02.12.2015 Bulletin 2015/49**

(21) Numéro de dépôt: **15174374.7**

(22) Date de dépôt: **14.12.2005**

(51) Int Cl.:
***C07K 16/28*** $^{(2006.01)}$ ***A61K 39/395*** $^{(2006.01)}$
***A61P 35/02*** $^{(2006.01)}$ ***A61P 37/00*** $^{(2006.01)}$

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **15.12.2004 FR 0413320**

(83) **Déclaration conformément à la règle 32(1) CBE (solution de l'expert)**

(62) Numéro(s) de document de la (des) demande(s) initiale(s) en application de l'article 76 CBE:
**05825974.8 / 1 824 887**

(71) Demandeur: **Laboratoire Français du Fractionnement et des Biotechnologies**
**91940 Les Ulis (FR)**

(72) Inventeurs:
- **DE ROMEUF, Christophe**
**59130 Lambersart (FR)**
- **GAUCHER, Christine**
**59320 Sequedin (FR)**
- **TEILLAUD, Jean-Luc**
**75004 Paris (FR)**
- **PROST, Jean-François**
**78000 Versailles (FR)**

(74) Mandataire: **Regimbeau**
**20, rue de Chazelles**
**75847 Paris Cedex 17 (FR)**

Remarques:
Cette demande a été déposée le 29-06-2015 comme demande divisionnaire de la demande mentionnée sous le code INID 62.

## (54) ANTICORPS CYTOTOXIQUE DIRIGE CONTRE LES PROLIFERATIONS HEMATOPOIETIQUES LYMPHOIDES DE TYPE B

(57) La présente invention se rapporte à un anticorps monoclonal dirigé contre l'antigène CD20, dont le domaine variable de chacune des chaînes légères est codé par la séquence d'acide nucléique murine SEQ ID NO : 5, le domaine variable de chacune des chaînes lourdes est codé par la séquence d'acide nucléique murine SEQ ID NO : 7, et les régions constantes des chaînes légères et des chaînes lourdes sont des régions constantes provenant d'une espèce non-murine, ainsi qu'à l'utilisation de cet anticorps pour activer les récepteurs Fc$\gamma$RIII de cellules immunitaires effectrices, et pour la fabrication d'un médicament, notamment destiné au traitement d'une leucémie ou d'un lymphome.

EP 2 949 674 A1

## Description

La présente invention se rapporte à un anticorps monoclonal dirigé contre l'antigène CD20, dont la région variable de chacune des chaînes légères est codée par une séquence possédant au moins 70% d'identité avec la séquence d'acide nucléique murine SEQ ID NO : 5, la région variable de chacune des chaînes lourdes est codée par une séquence possédant au moins 70% d'identité avec la séquence d'acide nucléique murine SEQ ID NO : 7, et les régions constantes des chaînes légères et des chaînes lourdes sont des régions constantes provenant d'une espèce non-murine, ainsi qu'à l'utilisation de cet anticorps pour activer les récepteurs FcγRIII de cellules immunitaires effectrices, et pour la fabrication d'un médicament, notamment destiné au traitement d'une leucémie ou d'un lymphome.

## Introduction et art antérieur

L'antigène CD20 est une protéine transmembranaire hydrophobe d'un poids moléculaire de 35-37 kDa présente à la surface des lymphocytes B matures (Valentine et al. 1987 Proc Natl Acad Sci U S A. 84 (22) : 8085-9 ; Valentine et al. 1989 J.Biol. Chem. 264 (19) :11282-11287). Il est exprimé au cours du développement des lymphocytes B dès le stade pré-B précoce et ce jusqu'à la différenciation en plasmocyte stade où cette expression disparaît. L' antigène CD20 est présent à la fois sur les lymphocytes B normaux et sur les cellules B malignes. Plus particulièrement, l'antigène CD20 est exprimé sur la plupart des lymphomes de phénotype B (80% des lymphomes) : il est exprimé par exemple sur plus de 90% des lymphomes non-Hodgkiniens (NHL) de lymphocytes B, et sur plus de 95% des Leucémies Lymphoides Chroniques de type B (LLC-B). L'antigène CD20 n'est pas exprimé sur les cellules souches hématopoïétiques ni sur les plasmocytes.

La fonction du CD20 n'est pas encore totalement élucidée, mais il pourrait agir comme un canal calcique et intervenir dans la régulation des premières étapes de la différenciation (Golay et al. 1985 J Immunol. ;135(6) :3795-801) et de la prolifération (Tedder et al.1986 Eur J Immunol. 1986 Aug;16(8):881-7) des lymphocytes B.

Ainsi, bien qu'il subsiste des incertitudes quant à son rôle dans l'activation et la prolifération des lymphocytes B, l'antigène CD20 est, de par sa localisation, une cible importante pour le traitement des pathologies impliquant des lymphocytes B tumoraux, comme par exemple les NHL ou la LLC-B par des anticorps reconnaissant spécifiquement le CD20. De plus, cet antigène est une cible idéale car il s'agit d'une protéine membranaire pour laquelle on ne connaît pas de modulation d'expression ou de polymorphisme.

Un seul anticorps monoclonal anti-CD20 non radiomarqué, le Rituxan® (Genentech), est actuellement disponible sur le marché pour le traitement des lymphomes de cellules B. Il montre chez les patients atteints de NHL des résultats cliniques encourageants lorsqu'il est associé à une chimiothérapie. Toutefois, son efficacité reste variable et souvent modeste lorsqu' il est utilisé comme agent unique (Teeling et al. 2004 Blood 104(6) :1793-800).

Par ailleurs, le Rituxan® n'a qu'un effet modeste sur les lymphocytes B de LLC-B. Cette faible efficacité est liée à différents phénomènes : d'une part, les lymphocytes B de LLC-B n'expriment le CD20 qu'en quantité relativement faible, d'autre part, le Rituxan® n'induit qu'une très faible activité ADCC (Antibody-Dépendent Cellular Cytotoxicity) vis à vis de ces cellules in vitro. Ces deux observations pourraient expliquer la corrélation constatée entre le degré d'expression du CD20 sur la tumeur (en cytométrie de flux quantitative) et la réponse au traitement.

La LLC-B étant la forme de leucémie la plus fréquente dans les pays occidentaux, avec des traitements lourds par chimiothérapie parfois insuffisants, comportant des effets secondaires qui aboutissent à une aplasie des cellules hématopoiétiques et à un déficit immunitaire, les anticorps monoclonaux apparaissent comme un recours innovant. Il est donc de première importance de mettre au point des anticorps capables de cibler spécifiquement l'antigène CD20 et permettant de détruire les cellules tumorales n'exprimant que faiblement cet antigène comme la LLC-B.

Les anticorps 2F2 et 7D8 proposés par Genmab (Teeling et al. 2004 Blood 104 (6):1793-800) pour traiter la LLC-B possèdent une capacité à activer le complément supérieure à celle engendrée par le Rituxan®. Toutefois, ces anticorps possèdent une activité ADCC faible, similaire au Rituxan®. Or, certains cliniciens montrent que l'activité complément est la cause d'effets secondaires délétères car les anticorps déclenchent alors un système d'activation conduisant à la production de molécules (notamment d'anaphylatoxines) ayant un large spectre d'activités non spécifiques (réactions inflammatoire, allergiques, vasculaires...). De plus, ces anticorps en sont encore au stade de la recherche, et leur efficacité clinique est encore à évaluer.

Le Demandeur, dans sa demande FR03/02713 (WO 2004/029092), décrit un anticorps anti-CD20, susceptible d'être produit dans la lignée YB2/0, sélectionné pour ses capacités à induire une forte ADCC et un fort taux de production d'IL-2 par la cellule Jurkat-CD16 en comparaison avec le Rituxan®. En effet, il existe un besoin important de nouveaux anticorps anti-CD20, permettant d' élargir la gamme de traitement de maladies des lymphocytes B par immunothérapie actuellement proposée, tout particulièrement pour les pathologies des lymphocytes B dans lesquelles l'antigène CD20 est faiblement exprimé sur les populations de lymphocytes B impliquées et pour lesquelles un traitement par immunothérapie satisfaisant n'existe pas.

C'est dans ce but que le Demandeur a mis au point de nouveaux anticorps anti-CD20, présentant une activité ADCC particulièrement élevée comparée au Rituxan®.

Description

Un premier objet de l'invention se rapporte ainsi à un anticorps monoclonal dirigé contre l'antigène CD20, dont la région variable de chacune des chaînes légères est codée par une séquence possédant au moins 70% d' identité avec la séquence d'acide nucléique murine SEQ ID NO : 5, la région variable de chacune des chaînes lourdes est codée par une séquence possédant au moins 70% d' identité avec la séquence d'acide nucléique murine SEQ ID NO : 7, et les régions constantes des chaînes légères et des chaînes lourdes sont des régions constantes provenant d'une espèce non-murine.

Les anticorps sont constitués de chaînes lourdes et de chaînes légères, liées entre elles par des ponts disulfures. Chaque chaîne est constituée, en position N-terminale, d'une région (ou domaine) variable (codée par les gènes réarrangés V-J pour les chaînes légères et V-D-J pour les chaînes lourdes) spécifique de l'antigène contre lequel l'anticorps est dirigé, et en position C-terminale, d'une région constante, constituée d'un seul domaine CL pour les chaînes légères ou de plusieurs domaines pour les chaînes lourdes.

Aux fins de l'invention, les expressions " anticorps monoclonal " ou " composition d'anticorps monoclonal " se réfèrent à une préparation de molécules d'anticorps possédant une spécificité identique et unique.

L'anticorps selon l'invention, dont les régions variables des chaînes légères et lourdes appartiennent à une espèce différente des régions constantes des chaînes légères et des chaînes lourdes, est qualifié d'anticorps " chimérique ".

Les séquences d'acide nucléique murines SEQ ID NO : 5 et SEQ ID NO : 7 codent pour le domaine variable de chacune des chaînes légères et le domaine variable de chacune des chaînes lourdes respectivement de l'anticorps produit par l'hybridome murin CAT-13.6E12, disponible à la Deutsche Sammlung von Mikroorganismen und Zellkulturen GmbH (DSMZ) sous le numéro ACC 474. Cet hybridome produit un anticorps monoclonal murin de type IgG2a,κ dirigé contre le CD20.

Ces séquences murines ont été choisies pour dériver les séquences des régions variables des anticorps selon l'invention en raison de la spécificité de l'anticorps murin CAT-13.6E12 pour l'antigène CD20, antigène également reconnu par Rituxan®. Les régions variables des anticorps selon l'invention comportent au moins 70% d' identité avec les séquences SEQ ID NO : 5 et SEQ ID NO: 7, cette identité de séquences conférant une identité de spécificité des anticorps selon l'invention avec l'anticorps murin CAT-13.6E12. De préférence, cette identité de séquences confère également une identité d'affinité pour la cible entre l'anticorps selon l'invention et l'anticorps murin CAT-13.6E12.

De plus, le Demandeur a montré de manière surprenante que l'anticorps murin CAT-13.6E12 possède la capacité d'induire la sécrétion d'IL-2 en présence de cellules Jurkat-CD16 exprimant les ectodomaines du récepteur FcγRIIIA (comme cela est illustré dans la Figure 11), indiquant une forte fixation de l'anticorps murin avec le CD16 FcγRIIIA humain, ce qui a encore motivé le Demandeur dans son choix.

Les anticorps selon l'invention possèdent en outre des régions constantes de ses chaînes légères et lourdes appartenant à une espèce non-murine. A cet égard, toutes les familles et espèces de mammifères non-murins sont susceptibles d'être utilisées, et en particulier l'homme, le singe, les muridés (sauf la souris), les suidés, les bovidés, les équidés, les félidés, les canidés, par exemple, ainsi que les oiseaux.

Les anticorps selon l'invention peuvent être construits en utilisant les techniques standard de l'ADN recombinant, bien connues de l'homme du métier, et plus particulièrement en utilisant les techniques de construction des anticorps " chimériques " décrites par exemple dans Morrison et al., Proc. Natl. Acad. Sci. U.S.A., 81, pp. 6851-55 (1984), où la technologie de l'ADN recombinant est utilisée pour remplacer la région constante d'une chaîne lourde et/ou la région constante d'une chaîne légère d'un anticorps provenant d'un mammifère non-humain avec les régions correspondantes d'une immunoglobuline humaine. Un mode de réalisation particulier sera illustré plus loin.

De manière avantageuse, la région variable de chacune des chaînes légères de l'anticorps selon l'invention est codée par une séquence possédant au moins 80% d'identité avec la séquence d'acide nucléique murine SEQ ID NO : 5, et la région variable de chacune des chaînes lourdes de l'anticorps selon l'invention est codée par une séquence possédant au moins 80% d'identité avec la séquence d'acide nucléique murine SEQ ID NO :7.

De manière avantageuse, la région variable de chacune des chaînes légères de l'anticorps selon l'invention est codée par une séquence possédant au moins 90% d'identité avec la séquence d'acide nucléique murine SEQ ID NO : 5, et la région variable de chacune des chaînes lourdes de l'anticorps selon l'invention est codée par une séquence possédant au moins 90% d'identité avec la séquence d'acide nucléique murine SEQ ID NO : 7.

De manière avantageuse, la région variable de chacune des chaînes légères de l'anticorps selon l'invention est codée par une séquence possédant au moins 95% d'identité avec la séquence d'acide nucléique murine SEQ ID NO : 5, et la région variable de chacune des chaînes lourdes de l'anticorps selon l'invention est codée par une séquence possédant au moins 95% d'identité avec la séquence d'acide nucléique murine SEQ ID NO : 7.

De manière avantageuse, la région variable de chacune des chaînes légères de l'anticorps selon l'invention est codée par une séquence possédant au moins 99% d'identité avec la séquence d'acide nucléique murine SEQ ID NO : 5, et la région variable de chacune des chaînes lourdes de l'anticorps selon l'invention est codée par une séquence possédant au moins 99% d'identité avec la séquence d'acide nucléique murine SEQ ID NO : 7.

Avantageusement, l'invention s'entend aussi de tout anticorps possédant des régions variables de leur chaînes lourdes et légères présentant une ou plusieurs substitution(s), insertion(s) ou délétion(s) d'un ou plusieurs acides nucléiques, ces modifications de séquences répondant aux pourcentages d' identités de séquences tels que définis ci-dessus, et n'altérant ni la spécificité de l'anticorps pour sa cible, ni son affinité pour sa cible.

Les anticorps de l'invention s'entendent aussi de tout anticorps possédant les régions CDR (Complementary Determining Region) de l'anticorps CAT-13.6E12, associées à des régions FR (framework, régions très conservées des régions variables, nommées aussi "charpente"). De tels anticorps possèdent une affinité et une spécificité très comparables, de préférence identiques, à l'anticorps murin CAT-13.6E12.

De manière préférée, la région variable de chacune des chaînes légères de l'anticorps selon l'invention est codée par la séquence d'acide nucléique murine SEQ ID NO : 5 ou par la séquence d'acide nucléique murine SEQ ID NO : 25, et la région variable de chacune des chaînes lourdes de l'anticorps selon l'invention est codée par la séquence d'acide nucléique murine SEQ ID NO : 7.

Dans un premier mode de réalisation de l'invention, un anticorps selon l'invention est donc un anticorps monoclonal dirigé contre l'antigène CD20, dont la région variable de chacune des chaînes légères est codée par la séquence d'acide nucléique murine SEQ ID NO : 5, la région variable de chacune des chaînes lourdes est codée par la séquence d'acide nucléique murine SEQ ID NO : 7, et les régions constantes des chaînes légères et des chaînes lourdes sont des régions constantes provenant d'une espèce non-murine.

Dans un deuxième mode de réalisation, l'anticorps selon l'invention est donc un anticorps monoclonal dirigé contre l'antigène CD20, dont la région variable de chacune des chaînes légères est codée par la séquence d'acide nucléique murine SEQ ID NO : 25, la région variable de chacune des chaînes lourdes est codée par la séquence d'acide nucléique murine SEQ ID NO : 7, et les régions constantes des chaînes légères et des chaînes lourdes sont des régions constantes provenant d'une espèce non-murine.

Les anticorps des deux modes de réalisation diffèrent dans leur séquence nucléotidique par un seul nucléotide : le nucléotide situé en position 318 des séquences SEQ ID NO : 5 et SEQ ID NO : 25, qui correspond respectivement à une cytosine et à une adénine.

Les anticorps de l'invention répondant à chacun de ces modes de réalisation possèdent une spécificité comparable, de préférence identique par rapport à l'antigène cible, le CD20, ainsi qu'une affinité comparable, de préférence identique par rapport au CD20.

De manière préférée, les régions constantes de chacune des chaînes légères et de chacune des chaînes lourdes de l'anticorps selon l'invention sont des régions constantes humaines. Ce mode de réalisation préféré de l'invention permet de diminuer l'immunogénicité de l'anticorps chez l'homme et par là même d'améliorer son efficacité lors de son administration thérapeutique chez l'homme.

Dans un mode de réalisation préféré de l'invention, la région constante de chacune des chaînes légères de l'anticorps selon l'invention est de type $\kappa$. Tout allotype convient à la réalisation de l'invention, par exemple Km(1), Km(1, 2), Km(1, 2, 3) ou Km(3) mais l'allotype préféré est Km(3).

Dans un autre mode de réalisation complémentaire, la région constante de chacune des chaînes légères de l'anticorps selon l'invention est de type $\lambda$.

Dans un aspect particulier de l'invention, et notamment lorsque les régions constantes de chacune des chaînes légères et de chacune des chaînes lourdes de l'anticorps selon l'invention sont des régions humaines, la région constante de chacune des chaînes lourdes de l'anticorps est de type $\gamma$. Selon cette variante, la région constante de chacune des chaînes lourdes de l'anticorps peut être de type $\gamma$1, de type $\gamma$2, de type $\gamma$3, ces trois types de régions constantes présentant la particularité de fixer le complément humain, ou encore de type $\gamma$4. Les anticorps possédant une région constante de chacune des chaînes lourdes de type $\gamma$ appartiennent à la classe des IgG. Les immunoglobulines de type G (IgG), sont des hétérodimères constitués de 2 chaînes lourdes et de 2 chaînes légères, liées entre elles par des ponts disulfures. Chaque chaîne est constituée, en position N-terminale, d'une région ou domaine variable (codée par les gènes réarrangés V-J pour la chaîne légère et V-D-J pour la chaîne lourde) spécifique de l'antigène contre lequel l'anticorps est dirigé, et en position C-terminale, d'une région constante, constituée d'un seul domaine CL pour la chaîne légère ou de 3 domaines (CH1, CH2 et CH3) pour la chaîne lourde. L'association des domaines variables et des domaines CH1 et CL des chaînes lourdes et légères forme les parties Fab, qui sont connectées à la région Fc par une région charnière très flexible permettant à chaque Fab de se fixer à sa cible antigénique tandis que la région Fc, médiatrice des propriétés effectrices de l'anticorps, reste accessible aux molécules effectrices telles que les récepteurs Fc$\gamma$R et le C1q. La région Fc, constituée des 2 domaines globulaires CH2 et CH3, est glycosylée au niveau du domaine CH2 avec la présence, sur chacune des 2 chaînes, d'un N-glycanne biantenné de type lactosaminique, lié à l'Asn 297.

De manière préférée, la région constante de chacune des chaînes lourdes de l'anticorps est de type $\gamma$1, car un tel anticorps montre une capacité à engendrer une activité ADCC chez le plus grand nombre d'individus (humains).

A cet égard, tout allotype convient à la réalisation de l'invention, par exemple G1m(3), G1m (1, 2, 17), G1m(1I, 17) ou G1m(1, 3) . De manière préférentielle, l'allotype est G1m(1, 17) .

Dans un aspect particulier de l'invention, la région constante de chacune des chaînes lourdes de l'anticorps est de type γ1, et elle est codée par la séquence d'acide nucléique humaine SEQ ID NO : 23, la région constante de chacune de ses chaînes légères étant codée par la séquence d'acide nucléique humaine SEQ ID NO : 21. Ainsi, un tel anticorps possède une région variable murine et une région constante humaine, avec des chaînes lourdes de type γ1. Cet anticorps appartient donc à la sous-classe des IgGl. Selon le mode de réalisation de l'anticorps selon l'invention, l'anticorps possède deux chaînes légères dont le domaine variable est codé par la séquence d' acide nucléique murine SEQ ID NO : 5 ou par la séquence d'acide nucléique murine SEQ ID NO : 25 et la région constante humaine est codée par la séquence d'acide nucléique SEQ ID NO : 21, et deux chaînes lourdes dont le domaine variable est codé par la séquence d'acide nucléique murine SEQ ID NO : 7 et la région constante est codée par la séquence d' acide nucléique humaine SEQ ID NO : 23.

De manière préférentielle, chacune des chaînes légères de l'anticorps selon l'invention est codée par la séquence d'acide nucléique chimère murine-humaine SEQ ID NO : 13 ou par la séquence d'acide nucléique chimère murine-humaine SEQ. ID NO : 27, et chacune des chaînes lourdes est codée par la séquence d'acide nucléique chimère murine-humaine SEQ ID NO : 19. La séquence d'acide nucléique chimère murine-humaine SEQ ID NO : 13 codant pour chacune des chaînes légères de l'anticorps est obtenue par fusion de la séquence d'acide nucléique murine SEQ ID NO : 5 codant pour le domaine variable de chacune des chaînes légères de l'anticorps et de la séquence d'acide nucléique humaine SEQ ID NO : 21 codant pour la région constante de chacune des chaînes légères de l'anticorps.

La séquence d'acide nucléique chimère murine-humaine SEQ ID NO : 27 codant pour chacune des chaînes légères de l'anticorps est obtenue par fusion de la séquence d'acide nucléique murine SEQ ID NO : 25 codant pour le domaine variable de chacune des chaînes légères de l'anticorps et de la séquence d'acide nucléique humaine SEQ ID NO : 21 codant pour la région constante de chacune des chaînes légères de l'anticorps.

La séquence d'acide nucléique chimère murine-humaine SEQ ID NO : 19 codant pour chacune des chaînes lourdes de l'anticorps est obtenue par fusion de la séquence d'acide nucléique murine SEQ ID NO : 7 codant pour le domaine variable de chacune des chaînes lourdes de l'anticorps et de la séquence d'acide nucléique humaine SEQ ID NO : 23 codant pour la région constante de chacune des chaînes lourdes de l'anticorps.

Dans un aspect particulier de l'invention, lorsque chacune des chaînes légères de l'anticorps est codée par la séquence d'acide nucléique chimère murine-humaine SEQ ID NO : 13 , et que chacune des chaînes lourdes est codée par la séquence d'acide nucléique chimère murine-humaine SEQ ID NO : 19, la séquence peptidique de chacune des chaînes légères, déduite de la séquence d'acide nucléique SEQ ID NO : 13, est la séquence SEQ ID NO : 14 et la séquence peptidique de chacune des chaînes lourdes, déduite de la séquence d'acide nucléique SEQ ID NO : 19, est la séquence SEQ ID NO : 20.

Dans un autre aspect particulier de l'invention, lorsque chacune des chaînes légères de l'anticorps est codée par la séquence d'acide nucléique chimère murine-humaine SEQ ID NO : 27, et que chacune des chaînes lourdes est codée par la séquence d'acide nucléique chimère murine-humaine SEQ ID NO : 19, la séquence peptidique de chacune des chaînes légères, déduite de la séquence d'acide nucléique SEQ ID NO : 27, est la séquence SEQ ID NO : 28 et la séquence peptidique de chacune des chaînes , lourdes, déduite de la séquence d'acide nucléique SEQ ID NO : 19, est la séquence SEQ ID NO : 20.

Les séquences peptidiques SEQ ID NO : 14 et SEQ ID NO : 28 diffèrent uniquement par l'acide aminé présent en position 106 de chacune de ces deux séquences. L'acide aminé situé en position 106 est une lysine (K) dans la séquence SEQ ID NO : 28 ; il s'agit d'une asparagine (N) dans la séquence SEQ ID NO : 14.

L'invention s'entend aussi des anticorps dont chacune des chaînes légères codées par une séquence d' acide nucléique chimère murine-humaine possède au moins 70% d'homologie ou d'identité avec la séquence d'acide nucléique chimère murine-humaine SEQ ID NO : 13 et chacune des chaînes lourdes codées par une séquence d' acide nucléique chimère murine-humaine possède au moins 70% d'homologie ou d'identité avec la séquence d' acide nucléique chimère murine-humaine SEQ ID NO : 19, ces modifications n'altérant ni la spécificité de l'anticorps ni ses activités effectrices, comme l'activité ADCC (Antibody-Dependent Cell-mediated Cytotoxicity) .

De manière particulièrement avantageuse, l'anticorps de l'invention est produit par une lignée cellulaire d'hybridome de rat. La lignée productrice de l'anticorps selon l'invention est une caractéristique importante puisqu'elle confère à l'anticorps certaines de ses propriétés particulières. En effet, le moyen d'expression des anticorps est à l'origine des modifications post-traductionnelles, notamment des modifications de la glycosylation, qui peuvent varier d'une lignée cellulaire à l'autre, et ainsi conférer des propriétés fonctionnelles différentes à des anticorps ayant pourtant des structures primaires identiques.

Dans un mode de réalisation préféré, l'anticorps est produit dans l'hybridome de rat YB2/0 (cellule YB2/3HL.P2.GI1 .16Ag.20, déposée à l'American Type Culture Collection sous le numéro ATCC CRL-1662). Cette lignée a été choisie en raison de sa capacité à produire des anticorps présentant une activité ADCC améliorée par rapport à des anticorps de même structure primaire produits par exemple dans CHO.

Dans un mode de réalisation particulier, un anticorps préféré selon l'invention est l'anticorps EMAB6 produit par le clone R509 déposé le 8 novembre 2004 sous le numéro d'enregistrement CNCM I-3314 à la Collection Nationale de Cultures de Microorganismes (CNCM, Institut Pasteur, 25 rue du Docteur Roux, 75724 Paris Cedex 15). Chacune des chaînes légères de l'anticorps EMAB6 est codée par la séquence d'acide nucléique chimère murine-humaine SEQ ID NO : 13, et chacune des chaînes lourdes est codée par la séquence d'acide nucléique chimère murine-humaine SEQ ID NO : 19. Cet anticorps chimérique entre en compétition avec l'anticorps murin CAT-13.6E12 pour la fixation du CD20 et possède une activité cytotoxique très supérieure à celle du Rituxan®, qui peut être attribuée pour partie à la glycosylation particulière du N-glycanne de la chaîne lourde de ces anticorps (cf. exemple 4). En effet, le clone R509 a pour particularité de produire une composition d' anticorps EMAB6 possédant un ratio taux de fucose/taux de galactose inférieur à 0,6, pour lequel il a été démontré dans la demande de brevet FR 03 12229 qu'il est optimal pour conférer une forte activité ADCC aux anticorps. Cet anticorps est donc particulièrement intéressant comme outil thérapeutique pour le traitement de pathologies dont les cellules à cibler expriment le CD20.

Dans un autre mode de réalisation particulier, un autre anticorps préféré selon l'invention est l'anticorps EMAB603 produit par le clone R603 déposé le 29 novembre 2005 sous le numéro d'enregistrement CNCM I-3529 à la Collection Nationale de Cultures de Microorganismes (CNCM, Institut Pasteur, 25 rue du Docteur Roux, 75724 Paris Cedex 15). Chacune des chaînes légères de l'anticorps EMAB603 est codée par la séquence d'acide nucléique chimère murine-humaine SEQ ID NO : 27, et chacune des chaînes lourdes est codée par la séquence d'acide nucléique chimère murine-humaine SEQ ID NO : 19. Cet anticorps chimérique entre en compétition avec l'anticorps murin CAT-13.6E12 pour la fixation du CD20 et possède une activité cytotoxique très supérieure à celle du Rituxan®, qui peut être attribuée pour partie à la glycosylation particulière du N-glycanne de la chaîne lourde de ces anticorps (cf. exemple 3). En effet, le clone R603 a pour particularité de produire une composition d'anticorps EMAB603 possédant un ratio taux de fucose/taux de galactose inférieur à 0,6 (cf exemple 4), pour lequel il a été démontré dans la demande de brevet FR 03 12229 qu' il est optimal pour conférer une forte activité ADCC aux anticorps. Cet anticorps est donc particulièrement intéressant comme outil thérapeutique pour le traitement de pathologies dont les cellules à cibler expriment le CD20.

Un autre objet de l'invention se rapporte au vecteur pEF-EMAB6-K d'expression de la chaîne légère d'un anticorps selon l'invention, de séquence SEQ ID NO : 12. Ce vecteur est le vecteur permettant l'expression d'un anticorps selon l'invention dont la chaîne légère est codée par la séquence d'acide nucléique SED ID NO : 13, dont la séquence peptidique déduite est la séquence SEQ ID NO : 14. Ce vecteur est une molécule d'acide nucléique dans laquelle la séquence d'acide nucléique murine SEQ ID NO : 5 codant pour le domaine variable de chacune des chaînes légères de l'anticorps et la séquence d'acide nucléique SEQ ID NO : 21 codant pour la région constante de chacune des chaînes légères de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à cette expression. De tels vecteurs sont bien connus de l'homme du métier, et peuvent être un adénovirus, un rétrovirus, un plasmide ou un bactériophage, cette liste n'étant pas limitative. De plus, toute cellule de mammifère peut être utilisée comme cellule hôte, c'est-à-dire comme cellule exprimant l'anticorps selon l'invention, par exemple YB2/0, CHO, CHO dhfr- (par exemple CHO DX B11, CHO DG44), CHO Lec13, SP2/0, NSO, 293, BHK ou COS.

Un autre objet de l'invention se rapporte au vecteur pEF-EMAB6-H d'expression de la chaîne lourde d'un anticorps selon l'invention, de séquence SEQ ID NO : 18. Ce vecteur est le vecteur permettant l'expression d'un anticorps selon l'invention dont la chaîne lourde est codée par la séquence d'acide nucléique SED ID NO : 19, dont la séquence peptidique déduite est la séquence SEQ ID NO : 20. Ce vecteur est une molécule d'acide nucléique dans laquelle la séquence d'acide nucléique murine SEQ ID NO : 7 codant pour le domaine variable de chacune des chaînes lourdes de l'anticorps et la séquence d'acide nucléique humaine SEQ ID NO : 23 codant pour la région constante de chacune des chaînes lourdes de l'anticorps ont été insérées, afin de les introduire et de les maintenir dans une cellule hôte. Il permet l'expression de ces fragments d'acide nucléique étrangers dans la cellule hôte car il possède des séquences indispensables (promoteur, séquence de polyadénylation, gène de sélection) à cette expression. Tout comme indiqué précédemment, le vecteur peut être par exemple un plasmide, un adénovirus, un rétrovirus ou un bactériophage, et la cellule hôte peut être toute cellule de mammifère, par exemple YB2/0, CHO, CHO dhfr- (CHO DX B11, CHO DG44), CHO Lec13, SP2/0, NS0, 293, BHK ou COS.

Un anticorps produit par co-expression des vecteurs pEF-EMAB6-K et pEF-EMAB6-H dans la cellule YB2/0 est illustré par l'anticorps anti-CD20 EMAB6, produit par le clone R509 (déposé sous le numéro d'enregistrement CNCM I-3314 à la CNCM). Cet anticorps induit une cytotoxicité supérieure à celle induite par Rituxan® à la fois sur les cellules de patients atteints de LLC-B (sur lesquelles l'antigène CD20 est plus faiblement exprimé) et sur des cellules Raji, utilisées en tant que modèle et exprimant le CD20 à une densité plus importante par rapport aux cellules de patients atteints de LLC-B. De plus, l'anticorps EMAB6 induit une sécrétion d'IL-2 (interleukine 2) par les cellules Jurkat-CD16 beaucoup plus forte que l'anticorps Rituxan®. L'anticorps EMAB6, pouvant être produit par culture du clone R509 dans un milieu de culture et à des conditions permettant l'expression des vecteurs précédemment décrits, est donc un outil des plus intéressants susceptibles de faire progresser la thérapie et le diagnostic des pathologies impliquant le CD20, et plus particulièrement la LLC-B, ainsi que la recherche dans ce domaine.

Un objet particulier de l'invention est une lignée cellulaire stable exprimant un anticorps selon 1' invention.

De manière avantageuse, la lignée cellulaire stable exprimant un anticorps selon l'invention est choisie parmi le groupe consistant en : SP2/0, YB2/0, IR983F, un myélome humain comme Namalwa ou toute autre cellule d'origine humaine comme PERC6, les lignées CHO, notamment CHO-K-1, CHO-Lec10, CHO-Lec1, CHO-Lec13, CHO Pro-5, CHO dhfr- (CHO DX B11, CHO DG44), ou d'autres lignées choisies parmi Wil-2, Jurkat, Vero, Molt-4, COS-7, 293-HEK, BHK, K6H6, NS0, SP2/0-Ag 14 et P3X63Ag8.653.

De manière préférée, la lignée utilisée est l'hybridome de rat YB2/0. Cette lignée a été choisie en raison de sa capacité à produire des anticorps présentant une activité ADCC améliorée par rapport à des anticorps de même structure primaire produits par exemple dans CHO.

Dans un aspect particulier de l'invention, la lignée cellulaire stable exprimant un anticorps selon l'invention, et plus particulièrement choisie dans le groupe précédemment décrit, a intégré les deux vecteurs d'expression pEF-EMAB6-K et pEF-EMAB6-H, tels que précédemment décrits.

Un aspect particulier de l'invention se rapporte au clone R509 déposé sous le numéro d'enregistrement CNCM I-3314 à la Collection Nationale de Cultures de Microorganismes (CNCM).

Un aspect particulier de l'invention se rapporte au clone R603 déposé sous le numéro d'enregistrement CNCM I-3529 à la Collection Nationale de Cultures de Microorganismes (CNCM).

Un autre objet de l'invention se rapporte à un fragment d'ADN de séquence SEQ ID NO : 19 codant pour la chaîne lourde d'un anticorps selon l'invention. La séquence d'acide nucléique chimère murine-humaine SEQ ID NO : 19 code pour chacune des chaînes lourdes de l'anticorps. Elle est obtenue par fusion de la séquence d'acide nucléique murine SEQ ID NO : 7 codant pour le domaine variable de chacune des chaînes lourdes de l'anticorps et de la séquence d'acide nucléique humaine SEQ ID NO : 23 codant pour la région constante de chacune des chaînes lourdes de l'anticorps.

Un autre objet particulier de l'invention se rapporte à un fragment d'ADN de séquence SEQ ID NO : 13 codant pour la chaîne légère d'un anticorps selon l'invention. La séquence d'acide nucléique chimère murine-humaine SEQ ID NO : 13 code pour chacune des chaînes légères de l'anticorps. Elle est obtenue par fusion de la séquence d'acide nucléique murine SEQ ID NO : 5 codant pour le domaine variable de chacune des chaînes légères de l'anticorps et de la séquence d'acide nucléique humaine SEQ ID NO : 21 codant pour la région constante de chacune des chaînes légères de l'anticorps.

Un autre objet particulier de l'invention se rapporte à un fragment d'ADN de séquence SEQ ID NO : 27 codant pour la chaîne légère d'un anticorps selon l'invention. La séquence d'acide nucléique chimère murine-humaine SEQ ID NO : 27 code pour chacune des chaînes légères de l'anticorps. Elle est obtenue par fusion de la séquence d'acide nucléique murine SEQ ID NO : 25 codant pour le domaine variable de chacune des chaînes légères de l'anticorps et de la séquence d'acide nucléique humaine SEQ ID NO : 21 codant pour la région constante de chacune des chaînes légères de l'anticorps.

Un objet particulier de l'invention se rapporte à l'utilisation de l'anticorps selon l'invention pour activer, in vivo ou in vitro, les récepteurs Fc$\gamma$RIIIA de cellules immunitaires effectrices. En effet, les anticorps de l'invention ont pour particularité et avantage d'être cytotoxiques. A ce titre, ils présentent la capacité d'activer par leur région Fc le récepteur Fc$\gamma$RIIIA. Ceci représente un intérêt considérable, car ce récepteur est exprimé à la surface de cellules appelées " cellules effectrices " : la liaison de la région Fc de l'anticorps à son récepteur porté par la cellule effectrice provoque l'activation du Fc$\gamma$RIIIA et la destruction des cellules cibles. Les cellules effectrices sont par exemple des cellules NK (Natural Killer), des macrophages, des neutrophiles, les lymphocytes CD8, les lymphocytes T$\gamma\delta$, les cellules NKT, les éosinophiles, les basophiles ou les mastocytes.

Dans un aspect particulier, l'anticorps de l'invention est utilisé comme médicament. Avantageusement, un tel médicament est destiné à traiter les maladies dont les cellules cibles sont des cellules exprimant le CD20, comme les lymphomes malins à cellules B.

Dans un aspect avantageux, l'anticorps selon l'invention est utilisé pour la fabrication d'un médicament destiné au traitement d'une leucémie ou d'un lymphome.

Un objet particulier de l'invention est l'utilisation de l'anticorps selon l'invention, pour la fabrication d'un médicament destiné au traitement d'une pathologie choisie parmi les leucémies aiguës lymphoblastiques B, les lymphomes lymphoblastiques B, les lymphomes à lymphocytes B matures, parmi lesquels la leucémie lymphoïde chronique de type B (LLC-B), le lymphome lymphocytique à petits lymphocytes B, la leucémie prolymphocytaire B, le lymphome lymphoplasmocytaire, le lymphome à cellules du manteau, le lymphome folliculaire, le lymphome de la zone marginale de type MALT, le lymphome ganglionnaire de la zone marginale avec ou sans cellule B monocytoïde, le lymphome splénique de la zone marginale (avec ou sans lymphocyte villeux), la leucémie à tricholeucocytes, le lymphome diffus à grandes cellules B, le lymphome de Burkitt ainsi que toute pathologie dysimmunitaire impliquant des cellules de la lignée lymphoïde B dont les maladies auto-immunes.

Un autre objet de l'invention est l'utilisation de l'anticorps selon l'invention pour la fabrication d'un médicament destiné au traitement d'une leucémie lymphoïde.

Un autre objet de l'invention est l'utilisation de l'anticorps selon l'invention pour la fabrication d'un médicament destiné au traitement de la leucémie lymphoïde chronique de type B (LLC-B). En outre, l'anticorps selon l'invention est particulièrement adapté à traiter des pathologies pour lesquelles le CD20 est plus faiblement exprimé sur les lymphocytes B, et de manière préférée la LLC-B (Leucémie Lymphoïde Chronique de type B). A cet égard, l'anticorps selon l'invention peut être utilisé en combinaison avec un ou plusieurs autre (s) anticorps, par exemple monoclonal (ux), dirigé (s) contre un ou plusieurs autre(s) antigène (s) exprimé (s) sur les cellules lymphoïdes, comme par exemple les antigènes HLA-DR, CD19, CD23, CD22, CD80, CD32 et CD52, pour la fabrication d'un médicament destiné au traitement d'une leucémie ou d'un lymphome. Ainsi, l'anticorps humanisé Campath-1H® (alentuzumab, MabCampathR®) qui cible une molécule abondamment exprimée sur les cellules lymphoïdes, l'antigène CD52, et permet d'induire une lyse cellulaire en mobilisant les mécanismes effecteurs de l'hôte fixation du complément, cytotoxicité dépendant des anticorps) est utilisé dans le traitement de la LLC (Moreton P, Hillmen P 2003 Semin. Oncol., 30(4) :493-501 ; Rawstron AC et al 2004 Blood, 103 (6) :2027-2031 ; Robak T 2004 Leuk. Lymphoma, 45(2) : 205-219 ; Stanglmaier M et al 2004 Ann Hematol., 83(10) :634-645). Des essais cliniques sont également en cours pour l'évaluation d'anticorps ou d'immunotoxines ciblant les antigènes HLA-DR, CD22, CD23, CD80 chez des patients atteints de LLC (Mavromatis BH, Cheson BD 2004 Blood Rev. 18 (2) : 137-148 ; Mavromatis B, Cheson BD 2003 J Clin. Oncol. 21(9) :1874-1881, Coleman M et al 2003 Clin. Cancer Res. 9 :3991S-3994S ; Salvatore G et al 2002 Clin. Cancer Res. 8:995-1002).

Dans un autre mode de réalisation, l'anticorps selon l'invention peut être utilisé en association avec des cellules exprimant des Fc$\gamma$R telles que les cellules NK, les cellules NKT (Natural Killers T), les lymphocytes T$\gamma\delta$, les macrophages, les monocytes ou les cellules dendritiques, c'est-à-dire en association avec une thérapie cellulaire (Peller S, Kaufman S Blood 1991, 78:1569, Platsoucas CD et al J Immunol 1982, 129:2305 ; Kimby E et al. 1989 Leukemia 3 (7) :501-504 ; Soorskaar D et al. 1988 Int Arch Allery Appl Immunol 87(2):159-164; Ziegler HW et al. 1981 Int J Cancer 27(3):321-327; Chaperot L et al. 2000 Leukemia 14 (9) :1667-77 , Vuillier F, Dighiero G 2003 Bull Cancer. 90(8-9) :744-50).

Par ailleurs, l'anticorps selon l'invention permet de manière avantageuse de réduire les doses administrées aux patients : avantageusement, la dose de l'anticorps administrée au patient est 2 fois, 5 fois, ou encore 10 fois, 25 fois, 50 fois ou de manière particulièrement avantageuse 100 fois moins élevée qu'une dose de Rituxan® .De manière avantageuse, la dose de l'anticorps administrée au patient est entre 2 et 5 fois, entre 5 et 10 fois, entre 5 et 25 fois, entre 5 et 50 fois ou encore entre 5 et 100 fois moins élevée qu'une dose de Rituxan®. Ainsi, l'anticorps selon l'invention, par exemple l'anticorps EMAB6, peut être administré avantageusement à une dose inférieure à 187,5 mg/m$^2$, à 75 mg/m$^2$, à 37,5 mg/m$^2$, 15 mg/m$^2$, 7,5 mg/m$^2$ ou de manière particulièrement avantageuse inférieure à 3,75 mg/m$^2$. La dose administrée est avantageusement comprise entre 187,5 mg/m$^2$ et 75 mg/m$^2$, ou entre 75 mg/m$^2$ et 37,5 mg/m$^2$, entre 75 mg/m$^2$ et 15 mg/m$^2$, entre 75 mg/m$^2$ et 7,5 mg/m$^2$ ou encore entre 75 mg/m$^2$ et 3,75 mg/m$^2$.

Ainsi, l'invention s'entend aussi d'une méthode de traitement des maladies dont les cellules cibles sont des cellules exprimant le CD20, comme les lymphomes malins à cellules B, comprenant l'administration à un patient, à une dose efficace, d'une composition comprenant un anticorps selon l'invention. Plus particulièrement, la méthode de traitement est particulièrement adaptée au traitement d'une leucémie ou d'un lymphome. De manière encore plus spécifique, il s'agit d'une méthode de traitement d'une pathologie choisie parmi les leucémies aiguës lymphoblastiques B, les lymphomes lymphoblastiques B, les lymphomes à cellules B matures, parmi lesquels la leucémie lymphoïde chronique de type B (LLC-B), le lymphome lymphocytique à petits lymphocytes B, la leucémie prolymphocytaire B, le lymphome lymphoplasmocytaire, le lymphome à cellules du manteau, le lymphome folliculaire, le lymphome de la zone marginale de type MALT, le lymphome ganglionnaire de la zone marginale avec ou sans cellule B monocytoïde, le lymphome splénique de la zone marginale (avec ou sans lymphocyte villeux), la leucémie à tricholeucocytes, le lymphome diffus à grandes cellules B, le lymphome de Burkitt ainsi que toute pathologie dysimmunitaire impliquant des cellules la lignée lymphoïde B dont les maladies auto-immunes, comprenant l'administration, à une dose efficace, d'un anticorps ou d'une composition comprenant l'anticorps selon l'invention.

Un objet particulier de l'invention est l'utilisation d'un anticorps selon l'invention pour la fabrication d'un médicament destiné au traitement de la maladie chronique du greffon contre l'hôte afin de traiter les manifestations impliquant les lymphocytes B du receveur. Enfin un dernier objet de l'invention est l'utilisation d'un anticorps selon l'invention pour la fabrication d'un médicament destiné au traitement du rejet de greffes d'organes, notamment de rein.

En effet, des études récentes (Ratanatharathorn et al. 2003 Biol Blood Marrow Transplant. 2003 Aug;9 (8) :505-11 ; Becker et al. 2004 Am J Transplant. 2004 Jun;4 (6) :996-1001) ont montré l'intérêt d'anticorps anti-CD20 dans le traitement de ces pathologies

D'autres aspects et avantages de l'invention seront décrits dans les exemples qui suivent, qui doivent être considérés comme illustratifs et ne limitent pas l'étendue de l'invention.

## Description des Figures

**Figure 1** : Représentation schématique du vecteur CKHu utilisé pour la chimérisation de la chaîne légère kappa des anticorps EMAB6 et EMAB603.

**Figure 2** : Représentation schématique du vecteur d'expression chaîne légère pEF-EMABG-K utilisé pour la production de l'anticorps EMAB6.

**Figure 3** : Représentation schématique du vecteur GlHu utilisé pour la chimérisation de la chaîne lourde des anticorps EMAB6 et EMAB603.

**Figure 4** : Représentation schématique du vecteur d'expression chaîne lourde pEF-EMAB6-H utilisé pour la production de l'anticorps EMAB6.

**Figure 5** : Compétition par l'anticorps chimérique EMAB6 pour la fixation de l'anticorps murin d'origine CAT-13.6E12 (CAT13) au CD20 exprimé sur les cellules Raji.

**Figure 6** : Activité cytotoxique complément dépendante des anticorps anti-CD20 sur les cellules Raji. **(A)** Rituxan® triangle vide, EMAB6 losange plein. La lyse des cellules est estimée par mesure de la LDH intracellulaire libérée dans le surnageant. Résultats exprimés en pourcentage de lyse, le 100% étant la valeur obtenue avec Rituxan® (à 5000ng/ml d'anticorps anti-CD20). Moyenne de 5 essais. (B) Comparaison des activités cytotoxiques complément dépendante des anticorps EMAB6 (losange plein) et EMAB603 (losange vide).

**Figure 7** : ADCC induite par les anticorps anti-CD20 vis à vis des cellules Raji. (A) Rituxan® triangle vide, EMAB6 losange plein. La lyse des cellules est estimée par mesure de la LDH intracellulaire libérée dans le surnageant. Résultats exprimés en pourcentage de lyse, le 100% étant la valeur obtenue avec Rituxan® (à 250ng/ml d'anticorps anti-CD20). Moyenne de 3 essais. (B) Comparaison de l'ADCC induite par les anticorps EMAB6 (losange plein) et EMAB603 (losange vide).

**Figure 8** : ADCC induite par les anticorps anti-CD20 en présence de lymphocytes B de patients présentant une LLC-B.
Rituxan® triangle vide, EMAB6 losange plein. Ratio E/T = 15. La lyse des cellules est estimée par mesure de la calcéine libérée dans le surnageant. Résultats exprimés en pourcentage, le 100% étant la valeur obtenue avec Rituxan® (à 500ng/ml d'anticorps anti-CD20). Moyenne de 4 expériences correspondant à 4 patients différents.

**Figure 9** : Activation du CD16 (FcγRIIIA) induite par les anticorps anti-CD20 en présence de cellules Raji. (A) Rituxan® triangle vide, EMAB6 losange plein. Résultats exprimés en pourcentage d'IL-2, celle-ci étant mesurée dans les surnageants par ELISA ; 100% étant la valeur obtenue avec Rituxan® (à 2500ng/ml d'anti-CD20). Moyenne de 4 essais. (B) Comparaison entre l'activation du CD16 (FcγRIIIA) induite par les anticorps EMAB6 (losange plein) et EMAB603 (losange vide).

**Figure 10** : Activation du CD16 (FcγRIIIA) induite par les anti-CD20 en présence de lymphocytes B de patients présentant une LLC-B. Rituxan® triangle vide, EMAB6 losange plein. Résultats exprimés en pourcentage d'IL-2, celle-ci étant mesurée dans les surnageants par ELISA ; 100% étant la valeur obtenue avec Rituxan® (à 2500ng/ml d'anticorps anti-CD20). Moyenne de 12 patients.

**Figure 11** : Production d'IL-2 induite par l'anticorps murin CAT-13.6E12 en présence des cellules Jurkat-CD16 (FcrRIIIA).

**Figure 12** : Représentation schématique du vecteur d'expression des chaînes lourde et légère pRSV-HL-EMAB603 utilisé pour la production de l'anticorps EMAB603.

## Exemples

### Exemple 1 : Construction des vecteurs d'expression des anticorps chimériques anti-CD20 EMAB6 et EMAB603

A. Détermination de la séquence des régions variables de l'anticorps murin CAT-13.6E12

L'ARN total de l'hybridome murin CAT-13.6E12 (fournisseur : DSMZ, réf. ACC 474) produisant une immunoglobuline de type IgG2a, κ a été isolé (kit RNAeasy, Qiagen réf. 74104) . Après transcription inverse, les domaines variables des chaînes légères (VK) et lourdes (VH)de l'anticorps CAT-13.6E12 ont été amplifiées par la technique de 5'RACE (Rapid Amplification of cDNA Ends) (kit GeneRacer, Invitrogen réf. L1500-01) . Les amorces utilisées pour ces deux étapes sont les suivantes :

1. amorces de transcription inverse

a. Amorce antisens spécifique Kappa murin (SEQ ID NO : 1)
5' - ACT GCC ATC AAT CTT CCA CTT GAC -3'
b. Amorce antisens spécifique G2a murin (SEQ ID NO : 2)
5'- CTG AGG GTG TAG AGG TCA GAC TG -3'

2. amorces de PCR 5'RACE

a. Amorce antisens spécifique Kappa murin (SEQ ID NO : 3)
5'- TTGTTCAAGAAGCACACGACTGAGGCAC -3'

b. Amorce antisens spécifique G2a murin (SEQ ID NO : 4)
5'- GAGTTCCAGGTCAAGGTCACTGGCTCAG -3'

Les produits de PCR VH et Vκ ainsi obtenus ont été clones dans le vecteur pCR4Blunt-TOPO (Zéro blunt TOPO PCR cloning kit, Invitrogen, réf. K2875-20) puis séquences.

La séquence nucléotidique de la région VA: de l'anticorps murin CAT-13.6E12 est indiquée sous la séquence SEQ ID NO : 5 et la séquence peptidique déduite est la séquence SEQ ID NO : 6. Le gène Vκ appartient à la famille Vκ4 [Kabat et al., "Séquences of Proteins of Immunological Interest", NIH Publication, 91-3242 (1991)].

La séquence nucléotidique de la région VH de CAT-13.6E12 est la séquence SEQ ID NO : 7 et la séquence peptidique déduite est la séquence SEQ ID NO : 8. Le gène VH appartient à la famille VH1 [Kabat et al., "Séquences of Proteins of Immunological Interest", NIH Publication, 91-3242 (1991)].

B. Construction des vecteurs d'expression chaîne lourde et chaîne légère des anticorps chimériques EMAB6 et EMAB603

1. Vecteur chaîne légère Kappa

1.1 Vecteur de chaîne légère de l'anticorps EMAB6

La séquence Vκ clonée dans le vecteur de séquençage pCR4Blunt-TOPO a été amplifiée à l'aide des amorces de clonage suivantes :

a) amorce sens Vκ (SEQ ID NO : 9) 5'- CTCAGTACTAGT**GCCGCCACC***ATG*GATTTTCAAGTGCAGATTTTCAG -3' La séquence soulignée correspond au site de restriction Spe I, la séquence en gras correspond à une séquence de Kozak consensus, l'ATG initiateur est en italiques.
b) amorce antisens VA: (SEQ ID NO : 10) 5'- **TGAAGACACTTGGTGCAGCCACAGT***CCGGTTTATTTCCAGCCTGGT* -3' Cette amorce réalise la jonction des séquences Vκ murine (en italique) et région constante (Cκ) humaine (en gras). La séquence soulignée correspond au site de restriction Dra III.

Le produit de PCR Vκ; ainsi obtenu contient la séquence codant le peptide signal naturel de l'anticorps murin CAT-13.6E12. Cette PCR Vκ a été ensuite clonée entre les sites Spe I et Dra III du vecteur de chimérisation chaîne légère (Fig. 1) qui correspond à la séquence SEQ ID NO : 11, en 5' de la région constante Cκ humaine, dont la séquence nucléique est la séquence SEQ ID NO : 21 et la séquence peptidique déduite est la séquence SEQ ID NO 22. La séquence Cκ humaine de ce vecteur de chimérisation avait été préalablement modifiée par mutagénèse silencieuse afin de créer un site de restriction Dra III pour permettre le clonage de séquences Vκ murines. Ce vecteur de chimérisation contient un promoteur RSV et une séquence de polyadénylation bGH (bovine Growth Hormone) ainsi que le gène de sélection dhfr (dihydrofolate reductase).

La séquence de la chaîne légère de l'anticorps chimérique EMAB6 codée par ce vecteur est présentée en SEQ ID NO : 13 pour la séquence nucléotidique et correspond à la séquence peptidique déduite SEQ ID NO : 14.

1.2 Vecteur de chaîne légère de l'anticorps EMAB603

Le protocole est le même que pour le vecteur de chaîne légère de l'anticorps EMAB6 (cf exemple 1, B-I.1), hormis l'amorce antisens Vκ qui est :

b' ) amorce antisens VK (SEQ ID NO : 29) 5'- **TGAAGACACTTGGTGCAGCCACAGT***CCGTTTATTTCCAGCCTGGT* -3' Cette amorce réalise la jonction des séquences VK murine (en italique) et région constante (CK) humaine (en gras). La séquence soulignée correspond au site de restriction Dra III.
Cette amorce introduit également la mutation AAC -> AAA (nucléotide encadré dans la séquence de l'amorce antisens SEQ ID NO : 29) qui correspond à la mutation N106K (cf. séquence nucléotidique et la séquence peptidique déduite SEQ ID NO : 25 et SEQ ID NO : 26) par rapport à la séquence naturelle Vκ de CAT-13.6E12 (cf. SEQ ID NO : 5 et SEQ ID NO : 6).

La séquence de la chaîne légère de l'anticorps chimérique EMAB603 codée par ce vecteur est présentée en SEQ ID NO : 27 pour la séquence nucléotidique et correspond à la séquence peptidique déduite SEQ ID NO : 28.

2. Vecteur chaîne lourde

Une démarche similaire a été appliquée pour la chimérisation de la chaîne lourde des anticorps EMAB6 et EMAB603.

La séquence VH clonée dans le vecteur pCR4Blunt-T0P0 a été tout d'abord amplifiée à l'aide des amorces de clonage suivantes :

a)amorce sens VH (SEQ ID NO :15) 5' - CTCAGTACTAGT**GCCGCCACC***ATG*GGATTCAGCAGGATCTTTCTC -3'
La séquence soulignée correspond au site de restriction Spe I, la séquence en gras correspond à une séquence de Kozak consensus, l'ATG initiateur est en italiques.
b) amorce antisens VH (SEQ ID NO :16) 5'- ***GACCGATGGGCCCTTGGTGGAGGC****TGAGGAGACGGTGACTGAGGTTCC* -3' Cette amorce réalise la jonction des séquences VH murine (en italique) et région constante G1 humaine (en gras). La séquence soulignée correspond au site de restriction Apa I.

Le fragment VH amplifié contient la séquence codant le peptide signal naturel de l'anticorps murin CAT-13.6E12. Cette PCR VH a été ensuite clonée entre les sites Spe I et Apa I du vecteur de chimérisation chaîne lourde (Fig. 3) qui correspond à la séquence SEQ ID NO : 17, en 5' de la région constante γ1 humaine dont la séquence nucléique est la séquence SEQ ID NO : 23 et la séquence peptidique déduite est la séquence SEQ ID NO : 24. Ce vecteur de chimérisation contient un promoteur RSV et une séquence de polyadénylation bGH (bovine Growth Hormone) ainsi que le gène de sélection néo.

La séquence de la chaîne lourde des anticorps chimériques EMAB6 et EMAB603 codée par ce vecteur est présenté en SEQ ID NO : 19 pour la séquence nucléotidique et en séquence SEQ ID NO : 20 pour la séquence peptidique déduite.

3. Vecteurs d'expression finaux

3.1 Vecteurs d'expression de l'anticorps EMAB6

Pour l'expression de l'anticorps EMAB6, le promoteur RSV du vecteur de chimérisation de la chaîne légère kappa (cf exemple. 1, B-1.1) a été remplacé par le promoteur EF-1 alpha humain. Le vecteur final d'expression chaîne légère pEF-EMAB6-K est présenté en Fig. 2 et correspond à la séquence SEQ ID NO : 12.

La séquence de la chaîne légère de l'anticorps chimérique EMAB6 codée par ce vecteur est présentée en SEQ ID NO : 13 pour la séquence nucléotidique et correspond à la séquence peptidique déduite SEQ ID NO :14.

Pour l'expression de l'anticorps EMAB6, le promoteur RSV du vecteur de chimérisation de la chaîne lourde (cf exemple 1, B-2) a été remplacé par le promoteur EF-1 alpha humain. Le vecteur final d'expression chaîne lourde pEF-EMAB6-H ainsi obtenu est présenté en Fig. 4 et correspond a la séquence SEQ ID NO : 18.

3.2 Vecteur d'expression de l'anticorps EMAB603

Un vecteur d'expression unique contenant les deux unités de transcription chaîne lourde et chaîne légère de l'anticorps anti-CD20 EMAB-603 a été construit à partir des deux vecteurs de chimérisation de la chaîne légère et de la chaîne lourde (cf respectivement exemple 1, B-1.2 et exemple 1-B2) par sous-clonage, dans le site Xho I du vecteur chaîne lourde, d'un fragment Bgl II-Pvu II du vecteur chaîne légère contenant l'unité de transcription chaîne légère et le gène dhfr. Ce vecteur d'expression pRSV-HL-EMAB-603 présente deux gènes de sélection néo (neo-phosphotransphérase II) et dhfr (dihydrofolate reductase) ainsi que deux unités de transcription chaîne lourde et chaîne légère sous le contrôle d'un promoteur RSV (Figure 12).

**Exemple 2 : Création de lignées cellulaires dérivées de la lignée YB2/0 productrices de l'anticorps chimérique anti-CD20 EMAB6 et de l'anticorps chimérique anti-CD20 EMAB603**

La lignée de rat YB2/0 (ATCC # CRL-1662) a été cultivée en milieu EMS (Invitrogen, réf. 041-95181M) contenant 5% de sérum de veau foetal (JRH Biosciences, réf. 12107). Pour la transfection, 5 millions de cellules ont été électroporés (électroporateur Biorad, modèle 1652077) en milieu Optimix (Equibio, réf. EKITE 1) avec 25 μg de vecteur chaîne légère, pEF-EMAB6-K (Fig. 2), linéarisé par Aat II, et 27 μg de vecteur chaîne lourde, pEF- EMAB6-H (Fig. 4), linéarisé par Sca I pour l'expression de l'anticorps EMAB6, ou avec le vecteur pRSV-HL-EMAB-603 pour l'expression de l'anticorps EMAB603. Les conditions d'électroporation appliquées étaient de 230 volts et 960 microfarads pour une cuvette de 0,5 ml. Chaque cuvette d'électroporation a été ensuite répartie sur 5 plaques P96 avec une densité de 5000 cellules/puits.

La mise en milieu sélectif RPMI (Invitrogen, ref 21875-034) contenant 5% de sérum dialyse (Invitrogen, réf. 10603-017), 500 μg/ml de G418 (Invitrogen, réf. 10131-027) et 25 nM de methotrexate (Sigma, réf. M8407), a été réalisée 3 jours après la transfection.

Les surnageants des puits de transfection résistants ont été criblés pour la présence d'immunoglobuline (Ig) chimérique par dosage ELISA spécifique des séquences Ig humaines.

Les 10 transfectants produisant le plus d'anticorps ont été amplifiés en plaques P24 et leur surnageant redosé par ELISA afin d'estimer leur productivité et de sélectionner les 3 meilleurs producteurs pour le clonage par dilution limite (40 cellules/plaque).

A l'issue du clonage, le clone R509.6A4 (R509-33903/046-6H1(1)6A4, productivité : 17 μg/$10^6$ cellules), dénommé ci-après « R509 », ainsi que le clone R603 ont été sélectionnés respectivement pour la production de l'anticorps chimérique EMAB6 et celle de l'anticorps EMAB603 et adaptés progressivement au milieu de production CD Hybridoma (Invitrogen, réf. 11279-023).

La production des anticorps chimériques EMAB6 et EMAB603 a été réalisée par expansion de la culture adaptée en milieu CD Hybridoma, obtenue par dilution à $3x10^5$ cellules/ml en flacons de 75 $cm^2$ et 175 $cm^2$ puis par dilution à $4,5x10^5$ cellules/ml en flacon de type roller. Après avoir atteint le volume maximal (1 1), la culture a été poursuivie jusqu'à ce que la viabilité cellulaire ne soit plus que de 20%. Après production, les anticorps chimériques EMAB6 et EMAB603 ont été purifiés par chromatographie d'affinité sur protéine A (pureté estimée par HPLC < 95 %) et contrôlés par électrophorèse en gel de polyacrylamide.

**Exemple 3 : Caractérisation de l'activité fonctionnelle des anticorps chimériques EMAB6 et EMAB603**

A. Spécificité

La spécificité de la reconnaissance antigénique de l'anticorps chimérique EMAB6 a été évaluée par une étude de compétition avec l'anticorps murin d'origine CAT-13.6E12 pour la fixation à l'antigène CD20 exprimé par les cellules Raji.

Pour cela, l'anticorps EMAB6 (10 μl de 0,5 à 50μg/ml) a été incubé à 4°C avec une quantité fixe de l'anticorps murin CAT-13.6E12 (10μl à 5μg/ml) pendant 20 minutes en présence de cellules Raji (50μl à $4x10^6$/ml). Après lavage, un anticorps anti-IgG de souris couplé à la phycoérythrine (PE) a été ajouté aux cellules Raji de façon à détecter spécifiquement la fixation de l'anticorps murin CAT-13.6E12. Les MFI obtenues en présence des différentes concentrations de EMAB6 sont traduites en pourcentage, le 100% correspondant à la fixation aux cellules de CAT-13.6E12 en absence de l'anticorps EMAB6.

On obtient ainsi une courbe d'inhibition de la fixation de l'anticorps CAT-13.6E12 (CAT13) sur les cellules Raji en présence de concentrations croissantes de EMAB6 (Fig. 5).

Cette étude démontre que le processus de chimérisation n'a pas altéré la spécificité de l'anticorps EMAB6 qui entre bien en compétition avec l'anticorps murin parental CAT-13.6E12 pour la fixation du CD20 exprimé à la surface des cellules Raji.

La spécificité de reconnaissance antigénique de l'anticorps EMAB603 est comparable à celle de l'anticorps EMAB6.

B. Activité Cytotoxique Dépendante du Complément

L'activité cytotoxique dépendante du complément des anticorps EMAB6 et EMAB603 est étudiée avec les cellules Raji en présence de sérum de jeune lapin comme source de complément ; l'anticorps anti-CD20 chimérique Rituxan® est inclus dans un test pour comparaison.

Pour ce test les cellules Raji sont ajustées à $6x10^5$ cellules/ml en IMDM (Iscove's Modified Dulbecco's Medium) 5% SVF. Les anticorps sont dilués en IMDM+0,5% SVF. Le mélange réactionnel est composé de 50μl d'anticorps, 50μl de sérum de jeune lapin (dilution au 1/10 en IMDM 0,5% SVF du réactif Cedarlane CL 3441), 50μl de cellules cibles et 50μl de milieu IMDM 0,5% SVF. Les concentrations finales d'anticorps sont de 5 000, 1 250, 250 et 50 ng/ml. Un contrôle sans anticorps est inclus dans le test. Après 1h d'incubation à 37°C sous atmosphère enrichie en 5% CO2, les plaques sont centrifugées et les taux de LDH intracellulaire libérée dans le surnageant évalués par un réactif spécifique (Cytotoxicity Détection Kit 1 644 793).

Le pourcentage de lyse est estimé en utilisant une gamme de calibration obtenue avec différentes dilutions de cellules cibles lysées au triton X100 (2%), correspondant à 100, 50, 25 et 0% de lyse respectivement.

Les résultats présentés en Fig. 6 (A) indiquent que les anticorps EMAB6 et Rituxan® induisent tous deux une lyse des cellules Raji dépendante du complément. Néanmoins, l'activité complément de l'anticorps EMAB6 apparaît légèrement inférieure à celle de Rituxan®. Cette différence est plus importante aux faibles concentrations d'anticorps utilisées dans ce test. Ainsi, pour les concentrations de 50 et 250ng/ml, l'activité de l'anticorps EMAB6 est de l'ordre de 45% de celle de Rituxan®. Cette différence s'amenuise lorsque la concentration d'anticorps est augmentée, le pourcentage d'activité cytotoxique dépendante du complément de l'anticorps EMAB6 représentant 92% de celle de Rituxan® à la plus forte concentration testée, c'est à dire 5000ng/ml.

Cette activité cytotoxique dépendante du complément de l'anticorps EMAB6 plus faible que celle de Rituxan® peut être considérée comme un avantage car elle limite la toxicité potentielle de l'EMAB6 « in vivo » comparé à Rituxan® liée à l'activation de la voie classique du complément conduisant à la production de différentes molécules ayant des activités inflammatoires, allergiques et vasculaires indésirables.

L'activité complément de l'anticorps EMAB603 apparaît Figure 6 (B).

C. Activité ADCC

La cytotoxicité de l'anticorps chimérique EMAB6 a été évaluée en présence de cellules Raji ou bien de lymphocytes B de patients atteints de LLC. L'anticorps anti-CD20 chimérique Rituxan® est inclus dans les tests pour comparaison.

La technique de mesure d'ADCC en marquage calcéine utilisée est la suivante :

Les cellules NK sont isolées à partir des PBMC en utilisant la technique de séparation sur billes magnétiques (MACS) de chez Myltenyi. Les cellules NK sont lavées et resuspendues en IMDM+5% SVF (45x$10^5$ cellules/ml). Les cellules effectrices et les cellules cibles sont utilisées dans un ratio 15/1. Les cellules Raji ou les PBMC (Peripheral Blood Mononuclear Cells) de patients présentant une LLC-B obtenues après Ficoll (>95% de lymphocytes B) sont préalablement marquées à la calcéine (1ml de cellules à 3x$10^6$ cellules/ml en IMDM +5% SVF + 20μl calcein (20μM) incubation 20min à 37°C puis lavage en HBSS (Hank's Buffered Saline Solution)) et ajustées à 3x$10^5$ cellules/ml en IMDM 5% SVF. Les anticorps sont dilués en IMDM 0,5% SVF (concentration finale 500 ; 50 ; 5 ; 0,5, ; 0,005 et 0,005 ng/ml) .

Le mélange réactionnel est composé de 50μl d'anticorps, 50μl de cellules effectrices, 50μl de cellules cibles et de 50μl de milieu IMDM en plaque de microtitration P96. Deux témoins négatifs sont établis :

- Lyse sans NK : les cellules effectrices NK sont remplacées par de l'IMDM + 5% SVF.
- Lyse sans anticorps (Ac) : les anticorps sont remplacées par de l'IMDM + 5% SVF.

Après 4h d'incubation à 37°C sous atmosphère enrichie en 5% $CO_2$, les plaques sont centrifugées et la fluorescence associée au surnageant mesurée sur un fluorimètre (excitation 485nm /émission 535nm).

Le pourcentage de lyse est estimé en utilisant une gamme de calibration obtenue avec différentes dilutions de cellules cibles lysées au Triton X100 (2%) correspondant à 100, 50, 25 et 0% de lyse respectivement.

Les résultats sont calculés dans un premier temps selon la formule suivante :

% de lyse = (%lyse avec Anticorps et NK) - (%lyse sans Anticorps) - (% de lyse sans NK), puis exprimés en pourcentage relatif, 100% étant la valeur obtenue à la plus forte concentration de Rituxan®.

Les résultats obtenus pour l'anticorps EMAB6 sur les cellules de la lignée Raji, présentés en Fig. 7 (A), montrent que quelle que soit la concentration testée, la cytotoxicité induite par l'anticorps EMAB6 est supérieure à celle induite par Rituxan®. Cette différence est particulièrement importante pour les faibles concentrations d'anticorps. Ainsi, à la concentration de 0,5ng/ml, les pourcentages de lyse sont de 96 et 4% pour EMAB6 et Rituxan® respectivement. En augmentant de 500 fois la dose (250ng/ml) la différence est encore appréciable puisque les pourcentages respectifs d'ADCC sont de 164 et 100% pour EMAB6 et Rituxan® respectivement. Lorsque l'on calcule les EC50 (concentration d'anticorps correspondant à 50% du E Max, efficacité maximale obtenue à la concentration d'anticorps la plus élevée et au plateau) par estimation graphique (en ng/ml) et selon l'hypothèse que Rituxan® et EMAB6 atteignent le même E Max, le rapport EC50 Rituxan®/EC50 EMAB6 dans ce test est alors égal à 300.

La cytotoxicité de l'anticorps chimérique EMAB603 a été évaluée en présence de cellules Raji selon le même procédé que pour l'anticorps EMAB6. Son activité est comparable à celle de l'anticorps EMAB6 (cf. Figure 7 (B)).

Avec les lymphocytes de patients présentant une LLC-B, les résultats obtenus, présentés en Fig. 8, démontrent que - quelle que soit la concentration testée, la cytotoxicité induite par l'anticorps EMAB6 est supérieure à celle induite par Rituxan®. Comme déjà observée avec les cellules Raji, cette différence est particulièrement importante pour les faibles concentrations d'anticorps. Une concentration de 0,5ng/ml de EMAB6 induit le même pourcentage de lyse que 500ng/ml de Rituxan® soit un rapport de concentrations de 1000. A la concentration de 5ng/ml, les pourcentages de lyse sont de 269 et 9% pour EMAB6 et Rituxan®, respectivement. A la dose maximale testée (500ng/ml), la différence est encore très importante puisque les pourcentages d'ADCC sont de 350 et 100% pour EMAB6 et Rituxan®, respectivement. Un résultat intéressant correspond aux concentrations qui donnent 50% de lyse. Le rapport EC50 Rituxan®/EC50 EMAB6 dans ce test est estimé à 10 000 (estimation graphique en ng/ml de EC50 selon l'hypothèse que Rituxan® et EMAB6 atteignent le même E Max).

Dans ces tests les activités cytotoxiques de EMAB6 et celle de EMAB603 sont donc très supérieures à celle de Rituxan®.

D. Activation du CD16 (sécrétion d'IL-2)

L'activation CD16 (FcγRIIIA) induite par l'anticorps chimérique EMAB6 a été déterminée en présence de cellules Raji ou bien de lymphocytes B de patients présentant une LLC. Ce test évalue la capacité de l'anticorps à se fixer sur le récepteur CD16 (FcγRIIIA) exprimé sur les cellules Jurkat-CD16 et à induire la sécrétion d'IL-2. L'anticorps anti-CD20 chimérique Rituxan® est inclus dans les tests pour comparaison.

La mesure d'activation CD16 est réalisée sur la lignée Jurkat-CD16 en présence de cellules Raji ou de lymphocytes B de patients présentant une LLC de la façon suivante :

Mélange en plaque de 96 puits : 50μl d'une solution d'anticorps (dilution à 10 000, 1 000, 100 et 10 ng/ml en IMDM 5% SVF pour les lymphocytes B de patients présentant une LLC-B et 10 000, 2 000, 1 000, 200, 100, 50 et 25 ng/ml pour les cellules Raji), 50μl de PMA (Phorbol Myristate Acétate, dilution à 40ng/ml en IMDM 5% SVF), 50μl de cellules Raji ou de PBMC de patients présentant une LLC-B obtenues après Ficoll (>95% de lymphocytes B) diluées à $6x10^5$/ml en IMDM 5% SVF, et 50μl de cellules Jurkat-CD16 (20x10E6/ml en IMDM 5% SVF) . Des contrôles sans anticorps sont inclus dans tous les tests.

Après incubation 1 nuit à 37°C, les plaques sont centrifugées, et l'IL-2 contenue dans les surnageants évaluée avec le kit commercial (Quantikine de chez R/D). La lecture de la DO se fait à 450nm.

Les résultats sont exprimés dans un premier temps en taux d' IL-2 en fonction de la concentration d'anticorps (de 0 à 2500ng/ml concentration finale), puis en pourcentage relatif, 100% étant la valeur obtenue avec Rituxan® à la plus forte concentration testée.

Les résultats obtenus avec les cellules de la lignée Raji, présentés en Fig. 9 (A), indiquent qu'en présence de l'anticorps EMAB6 et de Rituxan®, les cellules Jurkat-CD16 sécrètent de l'IL-2, indiquant une activation cellulaire via la fixation de la portion Fc des anticorps sur le CD16. Néanmoins l'anticorps EMAB6 possède une activité inductrice beaucoup plus forte que l'anticorps Rituxan®. Ainsi, à la concentration de 6,25ng/ml les pourcentages d'IL-2 sont de 112 et 21% pour EMAB6 et Rituxan® respectivement. A 50ng/ml, la différence est encore importante, les pourcentages d'IL-2 étant de 112 et 65% respectivement. Aux plus fortes concentrations, cette différence diminue, puisque à 2500ng/ml, les pourcentages respectifs d'IL-2 entre EMAB6 et Rituxan® sont de 124 et 100%. Le rapport EC50 Rituxan®/EC50 EMAB6 dans ce test est estimé à 15 (estimation graphique en ng/ml de EC50 selon l'hypothèse que Rituxan® et EMAB6 atteignent le même E Max).

Ces résultats confortent les résultats d'ADCC, tous deux dépendants du CD16. Ils indiquent que l'engagement du CD16 (FcγRIIIA) par la portion Fc de l'anticorps EMAB6 est suivie d'une forte activation cellulaire conduisant à l'induction de fonctions effectrices.

L'activation CD16 (FcγRIIIA) induite par l'anticorps chimérique EMAB603 en présence de cellules Raji est comparable avec celle induite par l'anticorps EMAB6.

Avec les lymphocytes de patients présentant une LLC-B, les résultats obtenus, présentés en Fig. 10, montrent qu'en présence des anticorps anti-CD20 Rituxan® et EMAB6, les cellules Jurkat-CD16 sécrètent de l'IL-2, indiquant une activation cellulaire via la fixation de la portion Fc des anticorps par le CD16. Néanmoins l'anticorps EMAB6 possède une capacité inductrice beaucoup plus importante que l'anticorps Rituxan®. En effet, l'activité d'induction de sécrétion d'IL-2 de Rituxan® est proche de la ligne de base pour les concentrations de 2,5 et 25ng/ml, alors que celle de l'anticorps EMAB6 est significative. Ainsi, à la concentration de 25ng/nl, les pourcentages d'IL-2 sont de 132 et 34% pour EMAB6 et Rituxan® respectivement. A la plus forte concentration (2500ng/ml), les pourcentages d'IL-2 sont de 148 et 100% respectivement. Le rapport EC50 Rituxan®/EC50 EMAB6 dans ce test est supérieur à 100 : il est estimé à 300 (estimation graphique en ng/ml de EC50 selon l'hypothèse que Rituxan® et EMAB6 atteignent le même E Max).

En conclusion, l'ensemble des tests effectués sur les cellules Raji démontre que les anticorps EMAB6 et EMAB603, contrairement à Rituxan® sont fortement cytotoxiques et induisent une activation des cellules exprimant le CD16 (FcγRIIIA) et cela particulièrement aux faibles concentrations d'anticorps. Par contre dans ces mêmes conditions, l'activité cytotoxique dépendante du complément de l'anticorps EMAB6 diminue d'environ 50% comparée à celle de Rituxan®.

Ces résultats sont renforcés par les études réalisées avec des cellules isolées à partir de patients présentant une LLC-B qui indiquent que l'anticorps EMAB6 est beaucoup plus cytotoxique que Rituxan® vis à vis des lymphocytes B de patients atteints de LLC-B. Les différences entre les deux anticorps sont plus marquées avec les cellules issues de patients atteints de LLC-B qu'avec les cellules Raji, démontrant un intérêt thérapeutique important pour EMAB6 comparé à Rituxan® dans cette pathologie.

L'origine de cette différence plus importante peut, entre autres, être due à la plus faible expression antigénique du CD20 sur les lymphocytes B de patients atteints de LLC-B par rapport à celle des cellules Raji.

Par analogie avec les cellules Raji, on peut suggérer que l'activité cytotoxique dépendante du complément de l'anticorps EMAB6 vis à vis des lymphocytes de patients atteints de LLC-B doit être inférieure à celle induite par Rituxan®, présentant ainsi l'avantage d'être moins toxique « in vivo » du fait des effets indésirables liés à une forte activation de la voie classique du complément.

**Exemple 4 : Analyse des glycannes de EMAB6 et EMAB603 par HPCE-LIF**

La structure du N-glycanne de la chaîne lourde des anticorps EMAB6 et EMAB603 a été analysée par HPCE-LIF. La structure du N-glycanne de la chaîne lourde de Rituxan® a aussi été analysée pour comparaison.

A cette fin les anticorps monoclonaux anti-CD20 ont été dessalés sur une colonne de Sephadex G-25 (HiTrap Desalting, Amersham Biosciences), séchés par évaporation et remis en suspension dans le tampon d'hydrolyse de la PNGase F (Glyko) en présence de 50 mM de *β*-mercaptoéthanol. Après 16 h d'incubation à 37°C, la fraction protéique a été précipitée par l'ajout d'éthanol absolu et le surnageant, qui contient les N-glycannes, séché par évaporation. Les oligosaccharides ainsi obtenus ont été soit marqués directement par un fluorochrome : l'APTS (1-amino-pyrène-3, 6, 8-trisulfonate), soit soumis à l'action d'exoglycosidases spécifiques avant marquage par l'APTS. Les oligosaccharides ainsi marqués ont été injectés sur un capillaire N-CHO, séparés et quantifiés par électrophorèse capillaire à détection de fluorescence induite par laser (HPCE-LIF).

L'évaluation du taux de fucose a été réalisée soit par l'addition des formes fucosylées isolées, soit plus spécifiquement après action simultanée de la neuraminidase, la β-galactosidase et la *N*-acétylhexosaminidase, permettant d'obtenir, sur 1' électrophorégramme, 2 pics correspondant au pentasaccharide [GlcNac2-Man3], fucosylé ou non:

Tableau 1 : analyse des N-glycannes des anticorps anti-CD20 EMAB603 et Rituxan®

| Anti-CD20 | % Fuco se | % Galactose | Fuc/G al |
|---|---|---|---|
| **EMAB60 3** | 15 | 37 | 0,4 |
| **Rituxa n®** | 93 | 57 | 1,63 |

Le taux de fucose, exprimé en %, a été calculé en utilisant la formule suivante :

$$\text{Taux de fucose} = \frac{\text{[GlcNac2-Man3] fucosylé x 100}}{\text{[GlcNac2-Man3+ GlcNac2-Man3 fucosylé]}}$$

Le taux de galactose, exprimé en %, a été calculé en additionnant les pourcentages des formes oligosaccharidiques contenant du galactose obtenues après action de la neuraminidase et de la fucosidase. La formule utilisée est la suivante :

$$\text{Taux de galactose} = (G1+G1B) + 2x(G2+ G2B)$$

Le rapport taux de fucose/taux de galactose est obtenu en divisant le taux de fucose par le taux de galactose, les taux étant calculés comme décrit ci-dessus.

D'après cette analyse (cf. Tableau 1), il apparaît que les anticorps EMAB6 et EMAB603 sont peu fucosylés (% Fucose inférieur à 25) comparé à Rituxan® (% Fucose =, 93) . De plus, le ratio Fuc/Gal (ratio taux de Fucose / taux de Galactose) de EMAB6 et de EMAB603 est faible (ratio Fuc/Gal inférieur à 0,6) contrairement aux anticorps exprimés dans CHO comme Rituxan (ratio Fuc/Gal = 1,63).

**Revendications**

1. Anticorps monoclonal dirigé contre l'antigène CD20, **caractérisé en ce que** la région variable de chacune de ses chaînes légères est codée par une séquence possédant au moins 70% d'identité avec la séquence d'acide nucléique murine SEQ ID NO : 5, la région variable de chacune de ses chaînes lourdes est codée par une séquence possédant au moins 70% d'identité avec la séquence d'acide nucléique murine SEQ ID NO : 7, et les régions constantes de ses chaînes légères et de ses chaînes lourdes sont des régions constantes provenant d'une espèce non-murine.

**2.** Anticorps selon la revendication 1, **caractérisé en ce que** ladite région variable de chacune de ses chaînes légères est codée par une séquence possédant au moins 80% d'identité avec la séquence d'acide nucléique murine SEQ ID NO : 5, et **en ce que** ladite région variable de chacune de ses chaînes lourdes est codée par une séquence possédant au moins 80% d'identité avec la séquence d'acide nucléique murine SEQ ID NO : 7.

**3.** Anticorps selon l'une quelconque des revendications 1 ou 2, **caractérisé en ce que** ladite région variable de chacune de ses chaînes légères est codée par une séquence possédant au moins 90% d'identité avec la séquence d'acide nucléique murine SEQ ID NO : 5, et **en ce que** ladite région variable de chacune de ses chaînes lourdes est codée par une séquence possédant au moins 90% d'identité avec la séquence d'acide nucléique murine SEQ ID NO : 7.

**4.** Anticorps selon l'une quelconque des revendications précédentes, **caractérisé en ce que** ladite région variable de chacune de ses chaînes légères est codée par une séquence possédant au moins 95% d'identité avec la séquence d'acide nucléique murine SEQ ID NO : 5, et **en ce que** ladite région variable de chacune de ses chaînes lourdes est codée par une séquence possédant au moins 95% d'identité avec la séquence d'acide nucléique murine SEQ ID NO : 7.

**5.** Anticorps selon l'une quelconque des revendications précédentes, **caractérisé en ce que** ladite région variable de chacune de ses chaînes légères est codée par une séquence possédant au moins 99% d'identité avec la séquence d'acide nucléique murine SEQ ID NO : 5, et **en ce que** ladite région variable de chacune de ses chaînes lourdes est codée par une séquence possédant au moins 99% d'identité avec la séquence d'acide nucléique murine SEQ ID NO : 7.

**6.** Anticorps selon l'une quelconque des revendications précédentes, **caractérisé en ce que** la région variable de chacune de ses chaînes légères est codée par la séquence d'acide nucléique murine SEQ ID NO : 5 ou par la séquence d'acide nucléique murine SEQ ID NO : 25, la région variable de chacune de ses chaînes lourdes est codée par la séquence d'acide nucléique murine SEQ ID NO : 7, et les régions constantes de ses chaînes légères et de ses chaînes lourdes sont des régions constantes provenant d'une espèce non-murine.

**7.** Anticorps selon l'une quelconque des revendications précédentes, **caractérisé en ce que** les régions constantes de chacune de ses chaînes légères et de chacune de ses chaînes lourdes sont des régions constantes humaines.

**8.** Anticorps selon l'une quelconque des revendications précédentes, **caractérisé en ce que** la région constante de chacune de ses chaînes légères est de type $\kappa$.

**9.** Anticorps selon l'une quelconque des revendications précédentes, **caractérisé en ce que** la région constante de chacune de ses chaînes lourdes est de type $\gamma$.

**10.** Anticorps selon la revendication 9, **caractérisé en ce que** la région constante de chacune de ses chaînes lourdes est de type $\gamma$1.

**11.** Anticorps selon la revendication 10, **caractérisé en ce que** la région constante de chacune de ses chaînes lourdes est de type y1 et codée par la séquence d'acide nucléique humaine SEQ ID NO : 23 et **en ce que** la région constante de chacune de ses chaînes légères est codée par la séquence d'acide nucléique humaine SEQ ID NO : 21.

**12.** Anticorps selon l'une quelconque des revendications précédentes, **caractérisé en ce que** chacune de ses chaînes légères est codée par la séquence d'acide nucléique chimère murine-humaine SEQ ID NO : 13 ou par la séquence d'acide nucléique chimère murine-humaine SEQ ID NO : 27 et **en ce que** chacune de ses chaînes lourdes est codée par la séquence d'acide nucléique chimère murine-humaine SEQ ID NO : 19.

**13.** Anticorps selon la revendication 12, **caractérisé en ce que** la séquence peptidique déduite de la séquence SEQ ID NO : 13 est la séquence SEQ ID NO : 14 et **en ce que** la séquence peptidique déduite de la séquence SEQ ID NO : 19 est la séquence SEQ ID NO : 20.

**14.** Anticorps selon la revendication 12, **caractérisé en ce que** la séquence peptidique déduite de la séquence SEQ ID NO : 27 est la séquence SEQ ID NO : 28 et **en ce que** la séquence peptidique déduite de la séquence SEQ ID NO : 19 est la séquence SEQ ID NO : 20.

**15.** Anticorps selon l'une quelconque des revendications précédentes, **caractérisé en ce qu'**il est produit par une lignée cellulaire d'hybridome de rat.

**16.** Anticorps selon la revendication 15, **caractérisé en ce qu'**il est produit dans l'hybridome de rat YB2/0 (cellule YB2/3HL.P2.G11.16Ag.20, déposée à l'American Type Culture Collection sous le numéro ATCC CRL-1662).

**17.** Anticorps selon l'une quelconque des revendications 1 à 13, **caractérisé en ce qu'**il s'agit de l'anticorps EMAB6 produit par le clone R509 déposé sous le numéro d'enregistrement CNCM I-3314 à la Collection Nationale de Cultures de Microorganismes (CNCM).

**18.** Anticorps selon l'une quelconque des revendications 1 à 12 et 14, **caractérisé en ce qu'**il s'agit de l'anticorps EMAB603 produit par le clone R603 déposé sous le numéro d'enregistrement CNCM I-3529 à la Collection Nationale de Cultures de Microorganismes (CNCM).

**19.** Vecteur pEF-EMAB6-K d'expression de la chaîne légère d'un anticorps selon l'une quelconque des revendications 1 à 13 de séquence SEQ ID NO : 12.

**20.** Vecteur pEF-EMAB6-H d'expression de la chaîne lourde d'un anticorps selon l'une quelconque des revendications 1 à 13 de séquence SEQ ID NO : 18.

**21.** Lignée cellulaire stable exprimant un anticorps selon l'une quelconque des revendications 1 à 18.

**22.** Lignée cellulaire stable selon la revendication 21 choisie parmi le groupe consistant en : SP2/0, YB2/0, IR983F, le myélome humain Namalwa, PERC6, les lignées CHO, notamment CHO-K-1, CHO-Lec10, CHO-Lec1, CHO-Lec13, CHO Pro-5, CHO dhfr-, Wil-2, Jurkat, Vero, Molt-4, COS-7, 293-HEK, BHK, K6H6, NS0, SP2/0-Ag 14 et P3X63Ag8.653.

**23.** Lignée cellulaire stable selon la revendication 21 ou 22, ayant intégré les deux vecteurs d'expression pEF-EMAB6-K et pEF-EMAB6-H selon les revendications 19 et 20.

**24.** Clone R509 déposé sous le numéro d'enregistrement CNCM I-3314 à la Collection Nationale de Cultures de Microorganismes (CNCM).

**25.** Clone R602 déposé sous le numéro d'enregistrement CNCM I-3529 à la Collection Nationale de Cultures de Microorganismes (CNCM).

**26.** Fragment d'ADN de séquence SEQ ID NO : 19 codant pour la chaîne lourde d'un anticorps selon l'une quelconque des revendications 1 à 18.

**27.** Fragment d'ADN de séquence SEQ ID NO : 13 codant pour la chaîne légère d'un anticorps selon l'une des revendications 1 à 13.

**28.** Fragment d'ADN de séquence SEQ ID NO : 27 codant pour la chaîne légère d'un anticorps selon l'une des revendications 1 à 12 et 14.

**29.** Utilisation d'un anticorps selon l'une quelconque des revendications 1 à 18, pour activer in vitro les récepteurs FcγRIII de cellules immunitaires effectrices.

**30.** Anticorps selon l'une quelconque des revendications 1 à 18, pour son utilisation comme médicament.

**31.** Utilisation d'un anticorps selon l'une quelconque des revendications 1 à 18, pour la fabrication d'un médicament destiné au traitement d'une leucémie ou d'un lymphome.

**32.** Utilisation d'un anticorps selon l'une quelconque des revendications 1 à 18, pour la fabrication d'un médicament destiné au traitement d'une pathologie choisie parmi les leucémies aiguës lymphoblastiques B, les lymphomes lymphoblastiques B, les lymphomes à cellules B matures, parmi lesquels la leucémie lymphoïde chronique de type B (LLC-B), le lymphome lymphocytique à petits lymphocytes B, la leucémie prolymphocytaire B, le lymphome lymphoplasmocytaire, le lymphome à cellules du manteau, le lymphome folliculaire, le lymphome de la zone marginale de type MALT, le lymphome ganglionnaire de la zone marginale avec ou sans cellule B monocytoïde, le lymphome splénique de la zone marginale (avec ou sans lymphocyte villeux), la leucémie à tricholeucocytes, le lymphome diffus à grandes cellules B, le lymphome de Burkitt, ainsi que toute pathologie dysimmunitaire impliquant des cellules de la lignée lymphoïde B dont les maladies auto-immunes.

**33.** Utilisation d'un anticorps selon l'une quelconque des revendications 1 à 18, pour la fabrication d'un médicament destiné au traitement d'une leucémie lymphoïde.

**34.** Utilisation d'un anticorps selon l'une quelconque des revendications 1 à 18, pour la fabrication d'un médicament destiné au traitement de la leucémie lymphoïde chronique de type B (LLC-B).

**35.** Utilisation d'un anticorps selon l'une quelconque des revendications 1 à 18, pour la fabrication d'un médicament destiné au traitement de la maladie du greffon contre l'hôte chronique.

**36.** Utilisation d'un anticorps selon l'une quelconque des revendications 1 à 18, pour la fabrication d'un médicament destiné au traitement du rejet de greffes d'organes, notamment de rein.

**37.** Utilisation d'un anticorps selon l'une quelconque des revendications 1 à 18 en combinaison avec un ou plusieurs autre(s) anticorps dirigé(s) contre un ou plusieurs autre(s) antigène(s) exprimé(s) sur les cellules lymphoïdes, pour la fabrication d'un médicament destiné au traitement d'une leucémie ou d'un lymphome.

**38.** Utilisation d'un anticorps selon la revendication 37, **caractérisée en ce que** ledit antigène exprimé sur les cellules lymphoïdes est choisi parmi le HLA-DR, le CD19, le CD23, le CD80, le CD22, le CD32 et le CD52.

**39.** Utilisation d'un anticorps selon l'une quelconque des revendications 1 à 18, en association avec des cellules exprimant des Fc$\gamma$R, telles que les cellules NK (Natural Killer), les cellules NKT (Natural Killers T), les lymphocytes T$\gamma\delta$, les macrophages, les monocytes ou les cellules dendritiques, pour la fabrication d'un médicament destiné au traitement d'une leucémie ou d'un lymphome.

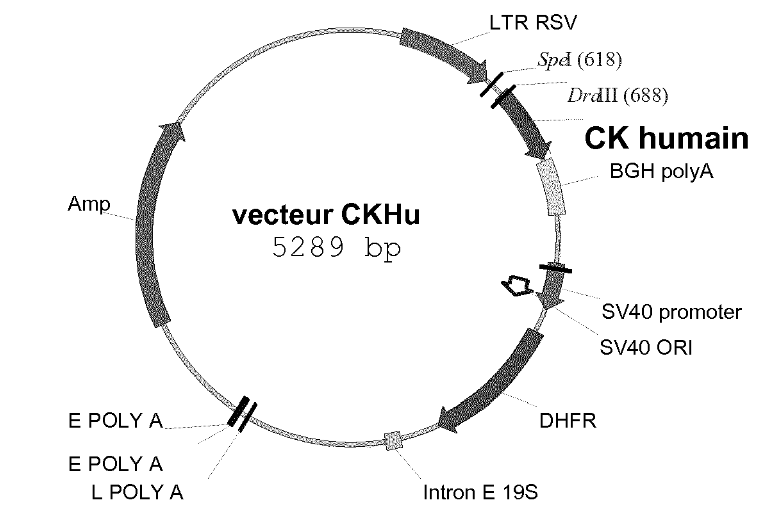

LTR RSV
SpeI (618)
DraIII (688)
CK humain
BGH polyA
Amp
vecteur CKHu
5289 bp
SV40 promoter
SV40 ORI
DHFR
E POLY A
E POLY A
L POLY A
Intron E 19S


**Fig. 1**

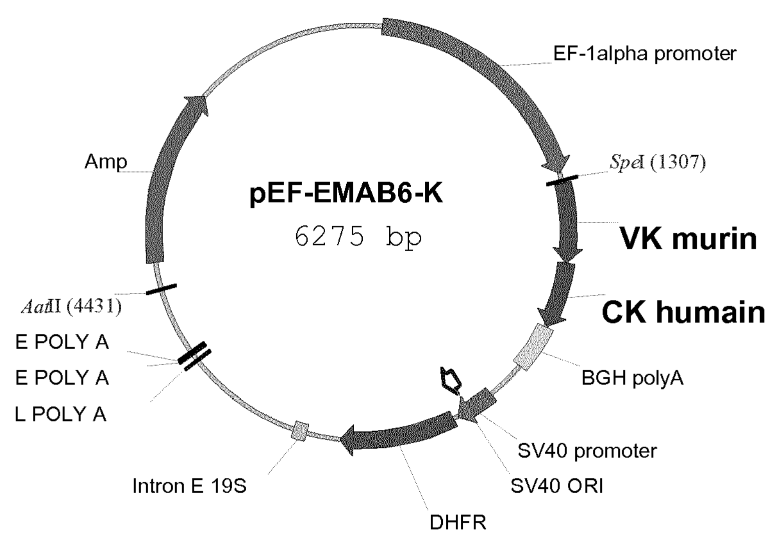

EF-1alpha promoter
SpeI (1307)
Amp
pEF-EMAB6-K
6275 bp
VK murin
CK humain
AatII (4431)
E POLY A
E POLY A
L POLY A
BGH polyA
SV40 promoter
SV40 ORI
Intron E 19S
DHFR


**Fig. 2**

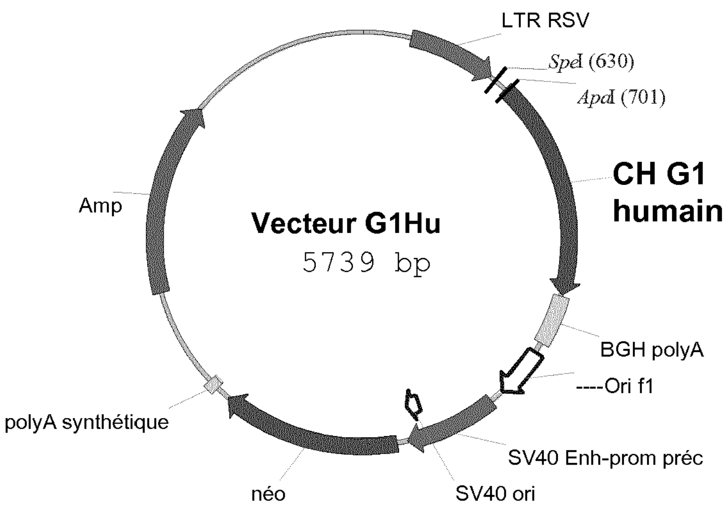
LTR RSV
SpeI (630)
ApaI (701)
CH G1
humain
Amp
Vecteur G1Hu
5739 bp
BGH polyA
----Ori f1
polyA synthétique
SV40 Enh-prom préc
néo
SV40 ori

Fig. 3

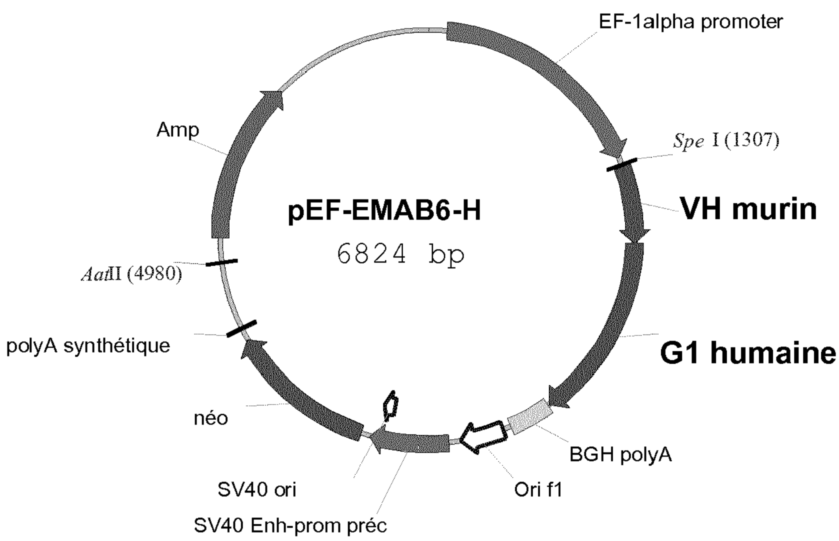
EF-1alpha promoter
Amp
Spe I (1307)
pEF-EMAB6-H
6824 bp
VH murin
AatII (4980)
polyA synthétique
G1 humaine
néo
BGH polyA
SV40 ori
Ori f1
SV40 Enh-prom préc

Fig. 4

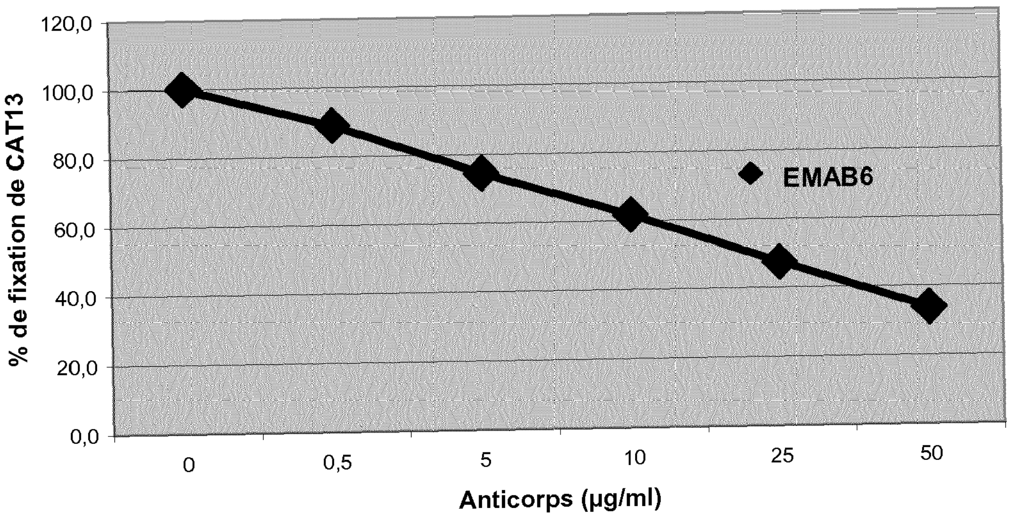
120,0
100,0
80,0
60,0
40,0
20,0
0,0
% de fixation de CAT13
EMAB6
0
0,5
5
10
25
50
Anticorps (µg/ml)

Fig. 5

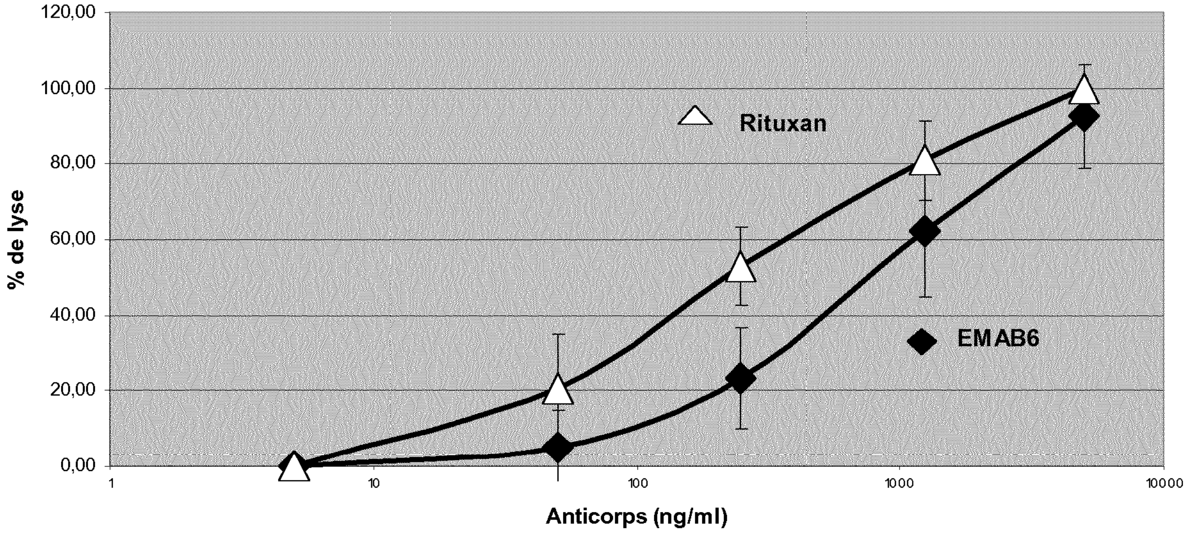
120,00
100,00
80,00
60,00
40,00
20,00
0,00
% de lyse
Rituxan
EMAB6
1
10
100
1000
10000
Anticorps (ng/ml)

Fig. 6 (A)

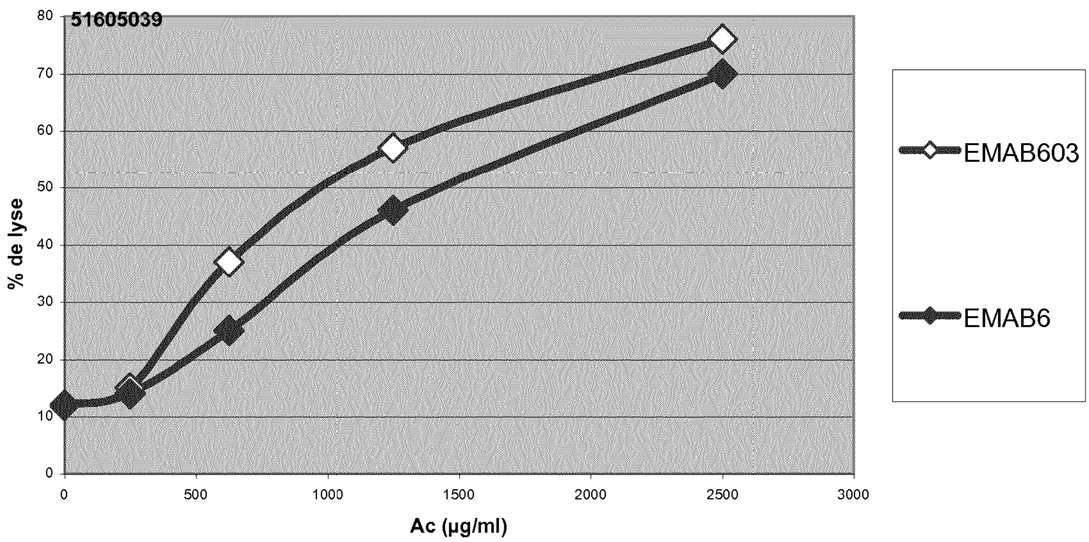
51605039
80
70
60
50
40
30
20
10
0
% de lyse
0
500
1000
1500
2000
2500
3000
Ac (µg/ml)
EMAB603
EMAB6

Fig. 6 (B)

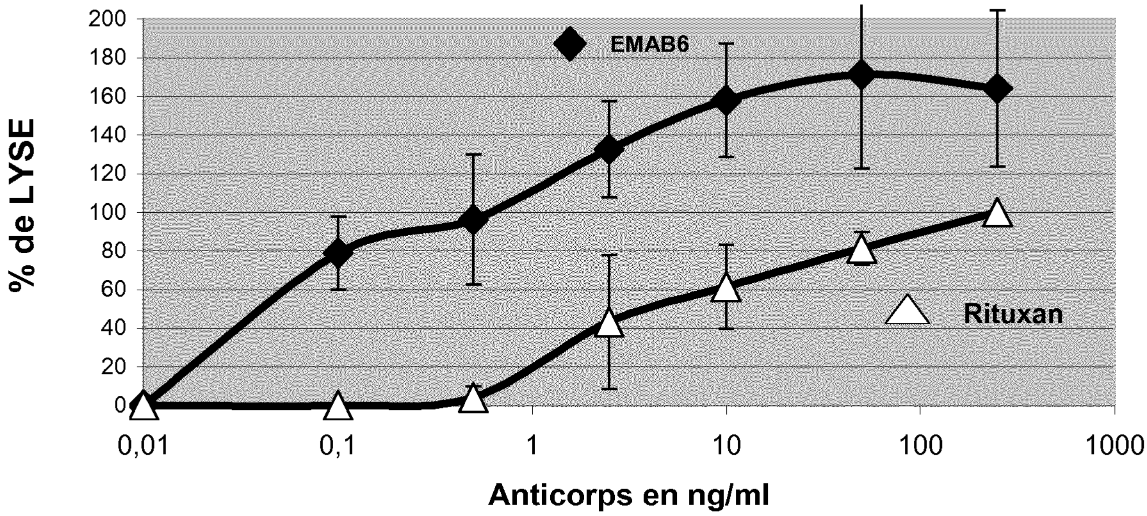
% de LYSE
200
180
160
140
120
100
80
60
40
20
0
0,01
0,1
1
10
100
1000
Anticorps en ng/ml
EMAB6
Rituxan

Fig. 7 (A)

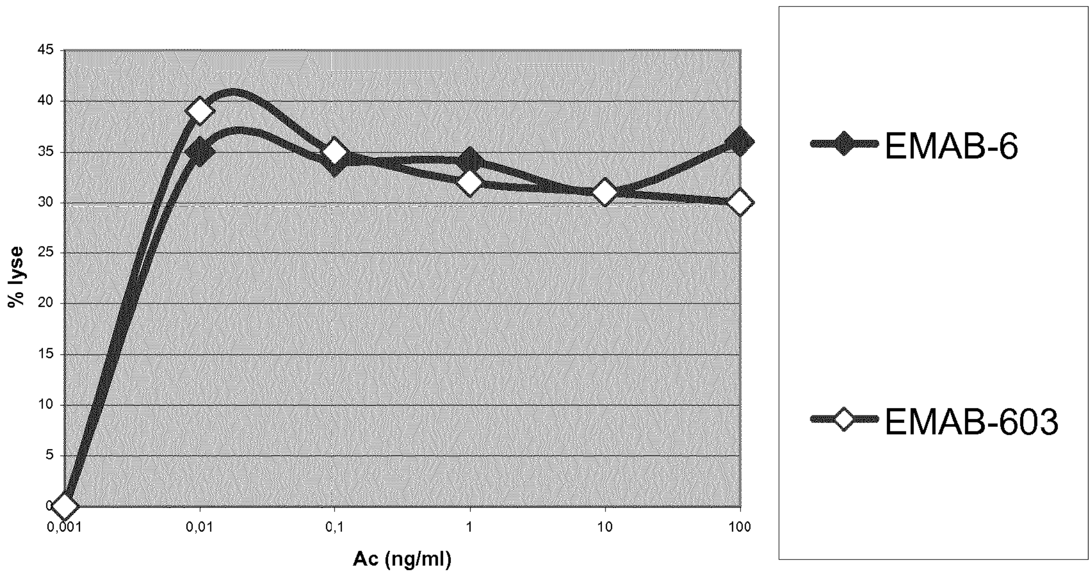
% lyse
45
40
35
30
25
20
15
10
5
0
0,001
0,01
0,1
1
10
100
Ac (ng/ml)
EMAB-6
EMAB-603

Fig.7 (B)

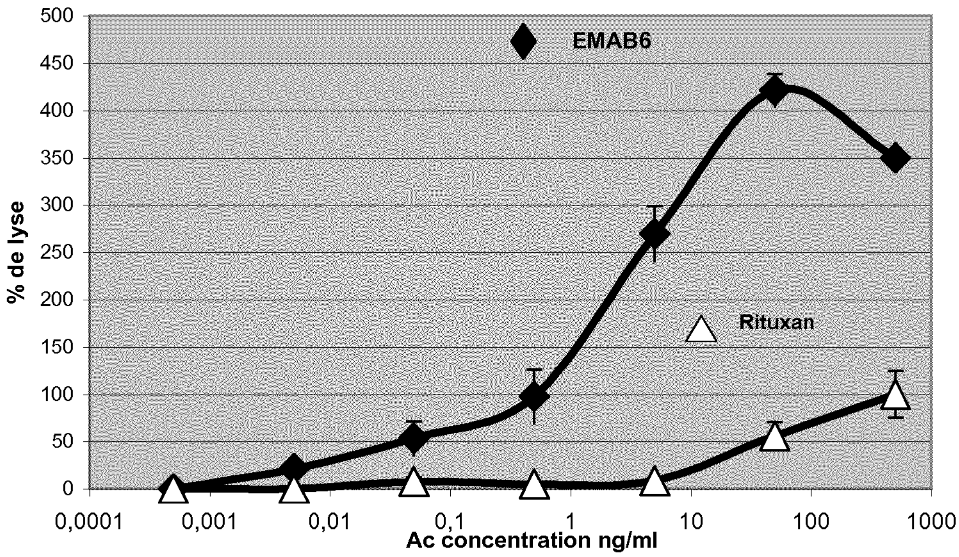
500
450
400
350
300
250
200
150
100
50
0
% de lyse
EMAB6
Rituxan
0,0001
0,001
0,01
0,1
1
10
100
1000
Ac concentration ng/ml

Fig. 8

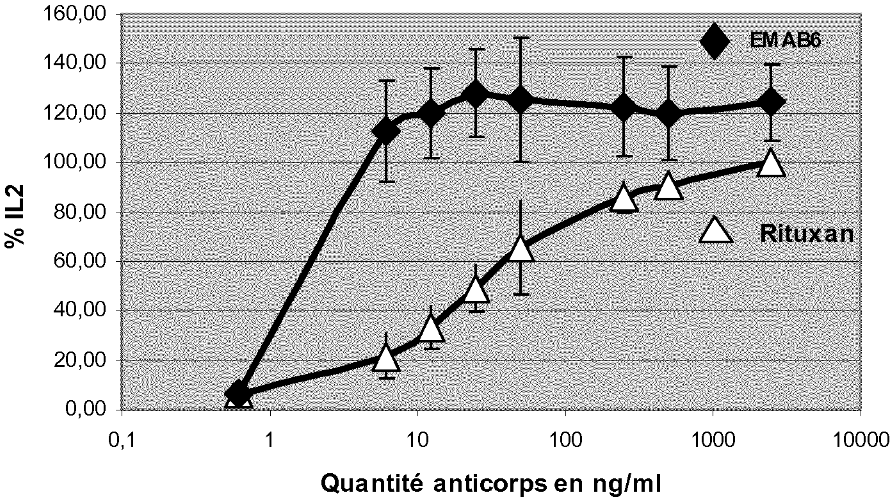
160,00
140,00
120,00
100,00
80,00
60,00
40,00
20,00
0,00
% IL2
0,1
1
10
100
1000
10000
Quantité anticorps en ng/ml
EMAB6
Rituxan

Fig. 9 (A)

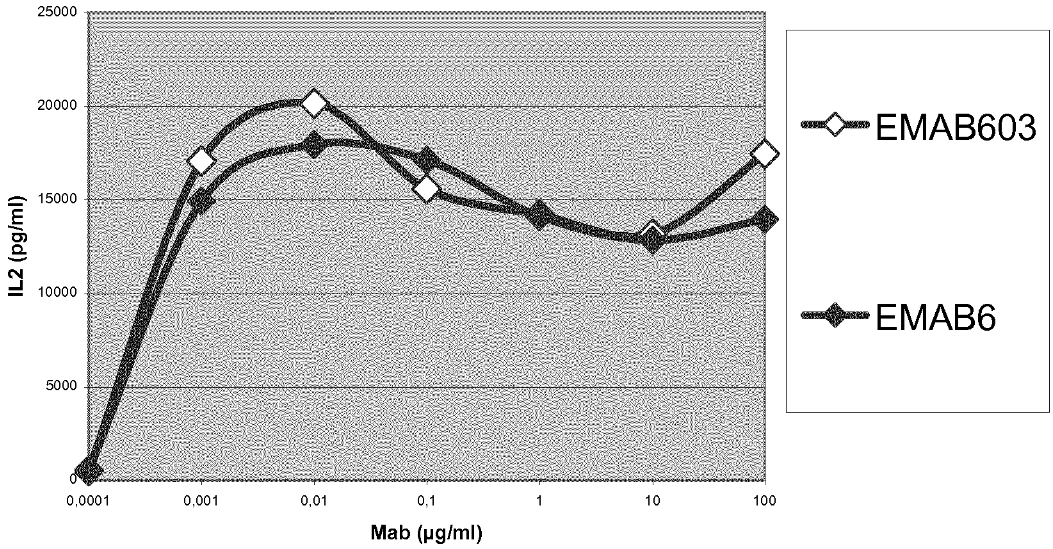
25000
20000
15000
10000
5000
0
IL2 (pg/ml)
0,0001
0,001
0,01
0,1
1
10
100
Mab (µg/ml)
EMAB603
EMAB6

Fig.9 (B)

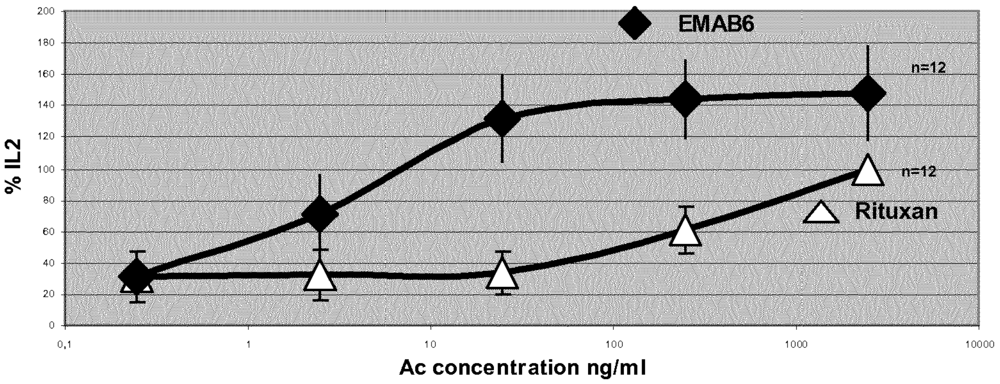
200
180
160
140
120
100
80
60
40
20
0
% IL2
EMAB6
n=12
n=12
Rituxan
0,1
1
10
100
1000
10000
Ac concentration ng/ml

Fig. 10

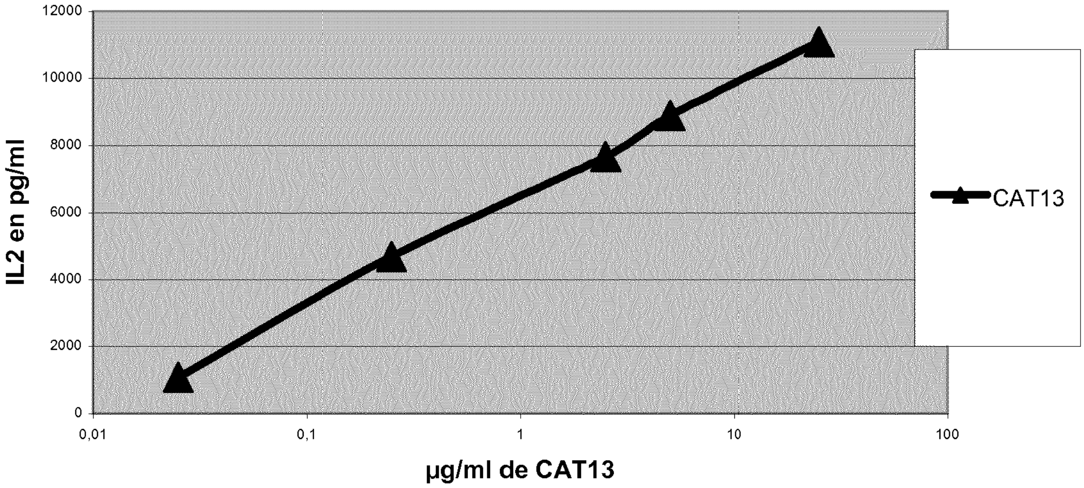
12000
10000
8000
6000
4000
2000
0
IL2 en pg/ml
0,01
0,1
1
10
100
µg/ml de CAT13
CAT13

Fig. 11

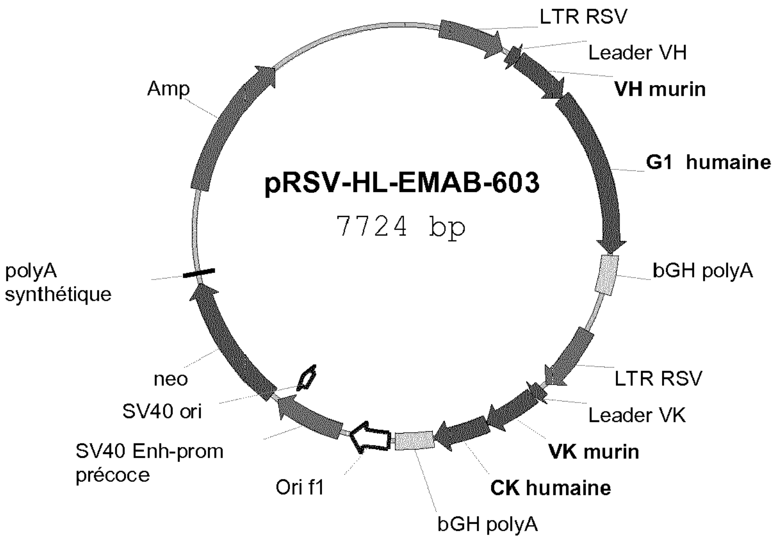
pRSV-HL-EMAB-603
7724 bp
LTR RSV
Leader VH
VH murin
G1 humaine
bGH polyA
LTR RSV
Leader VK
VK murin
CK humaine
bGH polyA
Ori f1
SV40 Enh-prom précoce
SV40 ori
neo
polyA synthétique
Amp

Fig.12

Europäisches Patentamt
European Patent Office
Office européen des brevets

# RAPPORT DE RECHERCHE EUROPEENNE

Numéro de la demande
EP 15 17 4374

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| Y | US 2003/133939 A1 (LEDBETTER JEFFREY A ET AL) 17 juillet 2003 (2003-07-17)<br>* pages 3-4; exemples 1-3 *<br>----- | 1-39 | INV.<br>C07K16/28<br>A61K39/395<br>A61P35/02<br>A61P37/00 |
| Y | WO 98/41645 A (IDEC PHARMACEUTICALS CORPORATION)<br>24 septembre 1998 (1998-09-24)<br>* figures 2A,9; exemple 7 *<br>----- | 1-39 | |
| Y | SHINKAWA T ET AL: "The absence of fucose but not the presence of galactose or bisecting N-acetylglucosamine of human IgG1 complex-type oligosaccharides shows the critical role of enhancing antibody-dependent cellular cytotoxicity",<br>JOURNAL OF BIOLOGICAL CHEMISTRY, AMERICAN SOCIETY OF BIOLOCHEMICAL BIOLOGISTS, BIRMINGHAM,, US,<br>vol. 278, no. 5,<br>31 janvier 2003 (2003-01-31), pages 3466-3473, XP002355427,<br>ISSN: 0021-9258<br>* le document en entier *<br>----- | 1-39 | |
| | | | DOMAINES TECHNIQUES RECHERCHES (IPC) |
| X,P | WO 2005/063815 A (BIOGEN IDEC MA INC; VAN VLIJMEN, HERMAN; TAYLOR, FREDERICK, R; GARBER,) 14 juillet 2005 (2005-07-14)<br>* page 37; exemple 4; séquences 3,5 *<br>-----<br>-/-- | 1-5 | C07K<br>C12N<br>A61K<br>A61P |

Le présent rapport a été établi pour toutes les revendications

1

| Lieu de la recherche | Date d'achèvement de la recherche | Examinateur |
|---|---|---|
| Munich | 9 octobre 2015 | Domingues, Helena |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..........................................................................................
& : membre de la même famille, document correspondant

EPO FORM 1503 03.82 (P04C02)

Europäisches Patentamt
European Patent Office
Office européen des brevets

## RAPPORT DE RECHERCHE EUROPEENNE

Numéro de la demande
EP 15 17 4374

| DOCUMENTS CONSIDERES COMME PERTINENTS | | | |
|---|---|---|---|
| Catégorie | Citation du document avec indication, en cas de besoin, des parties pertinentes | Revendication concernée | CLASSEMENT DE LA DEMANDE (IPC) |
| A | TEELING JESSICA L ET AL: "Characterization of new human CD20 monoclonal antibodies with potent cytolytic activity against non-Hodgkin lymphomas", BLOOD, vol. 104, no. 6, 15 septembre 2004 (2004-09-15), pages 1793-1800, XP002330734, ISSN: 0006-4971<br>* page 1794, colonne 1, alinéa 2 *<br>----- | 1-30 | |
| | | | DOMAINES TECHNIQUES RECHERCHES (IPC) |

Le présent rapport a été établi pour toutes les revendications

1

| Lieu de la recherche | Date d'achèvement de la recherche | Examinateur |
|---|---|---|
| Munich | 9 octobre 2015 | Domingues, Helena |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 15 17 4374

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-10-2015

| Document brevet cité au rapport de recherche | Date de publication | Membre(s) de la famille de brevet(s) | Date de publication |
|---|---|---|---|
| US 2003133939 A1 | 17-07-2003 | US 2003133939 A1 | 17-07-2003 |
| | | US 2005175614 A1 | 11-08-2005 |
| | | US 2005180970 A1 | 18-08-2005 |
| | | US 2005186216 A1 | 25-08-2005 |
| | | US 2005202012 A1 | 15-09-2005 |
| | | US 2005202023 A1 | 15-09-2005 |
| | | US 2005202028 A1 | 15-09-2005 |
| | | US 2005202534 A1 | 15-09-2005 |
| | | US 2005238646 A1 | 27-10-2005 |
| | | US 2011091461 A1 | 21-04-2011 |
| | | US 2011105729 A1 | 05-05-2011 |
| | | US 2013142793 A1 | 06-06-2013 |
| WO 9841645 A | 24-09-1998 | AT 296356 T | 15-06-2005 |
| | | AU 737155 B2 | 09-08-2001 |
| | | AU 6443598 A | 12-10-1998 |
| | | BR 9808584 A | 23-05-2000 |
| | | CA 2283740 A1 | 24-09-1998 |
| | | CN 1255166 A | 31-05-2000 |
| | | CZ 9903162 A3 | 16-02-2000 |
| | | DE 69830312 D1 | 30-06-2005 |
| | | DE 69830312 T2 | 02-02-2006 |
| | | EP 0981637 A1 | 01-03-2000 |
| | | ES 2242997 T3 | 16-11-2005 |
| | | ID 24565 A | 27-07-2000 |
| | | IN 189732 B | 19-04-2003 |
| | | JP 4128227 B2 | 30-07-2008 |
| | | JP 2001516221 A | 25-09-2001 |
| | | NO 994397 A | 09-11-1999 |
| | | PL 335695 A1 | 08-05-2000 |
| | | PT 981637 E | 30-09-2005 |
| | | RO 120148 B1 | 30-09-2005 |
| | | TW I232239 B | 11-05-2005 |
| | | US 6413777 B1 | 02-07-2002 |
| | | US 2002192820 A1 | 19-12-2002 |
| | | US 2004166528 A1 | 26-08-2004 |
| | | WO 9841645 A1 | 24-09-1998 |
| WO 2005063815 A | 14-07-2005 | US 2006275283 A1 | 07-12-2006 |
| | | US 2010203046 A1 | 12-08-2010 |
| | | WO 2005063815 A2 | 14-07-2005 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 0302713
- WO 2004029092 A
- FR 0312229


### Littérature non-brevet citée dans la description


- **VALENTINE et al.** *Proc Natl Acad Sci U S A.,* 1987, vol. 84 (22), 8085-9
- **VALENTINE et al.** *J.Biol. Chem.,* 1989, vol. 264 (19), 11282-11287
- **GOLAY et al.** *J Immunol.,* 1985, vol. 135 (6), 3795-801
- **TEDDER et al.** *Eur J Immunol.,* Août 1986, vol. 16 (8), 881-7
- **TEELING et al.** *Blood,* 2004, vol. 104 (6), 1793-800
- **MORRISON et al.** *Proc. Natl. Acad. Sci. U.S.A.,* 1984, vol. 81, 6851-55
- **MORETON P ; HILLMEN P.** *Semin. Oncol.,* 2003, vol. 30 (4), 493-501
- **RAWSTRON AC et al.** *Blood,* 2004, vol. 103 (6), 2027-2031
- **ROBAK T.** *Leuk. Lymphoma,* 2004, vol. 45 (2), 205-219
- **STANGLMAIER M et al.** *Ann Hematol.,* 2004, vol. 83 (10), 634-645
- **MAVROMATIS BH ; CHESON BD.** *Blood Rev.,* 2004, vol. 18 (2), 137-148
- **MAVROMATIS B ; CHESON BD.** *J Clin. Oncol.,* 2003, vol. 21 (9), 1874-1881
- **COLEMAN M et al.** *Clin. Cancer Res.,* 2003, vol. 9, 3991S-3994S
- **SALVATORE G et al.** *Clin. Cancer Res.,* 2002, vol. 8, 995-1002
- **PELLER S ; KAUFMAN S.** *Blood,* 1991, vol. 78, 1569
- **PLATSOUCAS CD et al.** *J Immunol,* 1982, vol. 129, 2305
- **KIMBY E et al.** *Leukemia,* 1989, vol. 3 (7), 501-504
- **SOORSKAAR D et al.** *Int Arch Allery Appl Immunol,* 1988, vol. 87 (2), 159-164
- **ZIEGLER HW et al.** *Int J Cancer,* 1981, vol. 27 (3), 321-327
- **CHAPEROT L et al.** *Leukemia,* 2000, vol. 14 (9), 1667-77
- **VUILLIER F ; DIGHIERO G.** *Bull Cancer.,* 2003, vol. 90 (8-9), 744-50
- **RATANATHARATHORN et al.** *Biol Blood Marrow Transplant,* Août 2003, vol. 9 (8), 505-11
- **BECKER et al.** *Am J Transplant.,* Juin 2004, vol. 4 (6), 996-1001
- **KABAT et al.** Séquences of Proteins of Immunological Interest. NIH Publication, 1991, 91-3242

SEQUENCE LISTING

<110> Laboratoire Français du Fractionnement et des Biotechnologies

<120> ANTICORPS CYTOTOXIQUE DIRIGE CONTRE LES PROLIFERATIONS HEMATOPOÏETIQUES LYMPHOÏDES DE TYPE B

<130> B72136D34848

<160> 29

<170> PatentIn version 3.1

<210> 1
<211> 24
<212> DNA
<213> Artificial Sequence

<220>
<223> Primer

<400> 1
actgccatca atcttccact tgac 24

<210> 2
<211> 23
<212> DNA
<213> Artificial Sequence

<220>
<223> Primer

<400> 2
ctgagggtgt agaggtcaga ctg 23

<210> 3
<211> 28
<212> DNA
<213> Artificial Sequence

<220>
<223> Primer

<400> 3
ttgttcaaga agcacacgac tgaggcac 28

<210> 4
<211> 28
<212> DNA
<213> Artificial Sequence

<220>
<223> Primer

<400> 4
gagttccagg tcaaggtcac tggctcag 28

<210> 5
<211> 321

<212> DNA
<213> Mus musculus

<400> 5
caaattgttc tctcccagtc tccagcaatc ctgtctgcat ctccagggga gaaggtcaca 60

atgacttgca gggccagctc aagtgtaagt tacatgcact ggtaccagca gaagccagga 120

tcctccccca aaccctggat ttatgccaca tccaacctgg cttctggagt ccctgctcgc 180

ttcagtggca gtgggtctgg gacctcttat tctttcacaa tcagcagagt ggaggctgaa 240

gatgctgcca cttattactg ccagcagtgg acttttaacc cacccacgtt cggagggggg 300

accaggctgg aaataaaccg g 321

<210> 6
<211> 107
<212> PRT
<213> Mus musculus

<400> 6

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Lys Pro Gly Ser Ser Pro Lys Pro Trp Ile Tyr
35 40 45

Ala Thr Ser Asn Leu Ala Ser Gly Val Pro Ala Arg Phe Ser Gly Ser
50 55 60

Gly Ser Gly Thr Ser Tyr Ser Phe Thr Ile Ser Arg Val Glu Ala Glu
65 70 75 80

Asp Ala Ala Thr Tyr Tyr Cys Gln Gln Trp Thr Phe Asn Pro Pro Thr
85 90 95

Phe Gly Gly Gly Thr Arg Leu Glu Ile Asn Arg
100 105

<210> 7
<211> 354
<212> DNA
<213> Mus musculus

<400> 7
caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggaggt atttatccag gaaatggtga tacttcctac 180

aatcagaagt tcaagggcaa ggccacactg actgtaggca aatcctccag cacagcctac 240

atgcagctca gcagcctgac atctgaagac tctgcggtct atttctgtgc aagatatgac 300

tacaactatg ctatggacta ctggggtcaa ggaacctcag tcaccgtctc ctca 354

<210> 8
<211> 118
<212> PRT
<213> Mus musculus

<400> 8

Gln Ala Tyr Leu Gln Gln Ser Gly Ala Glu Leu Val Arg Pro Gly Ala
1 5 10 15

Ser Val Lys Met Ser Cys Lys Ala Ser Gly Tyr Thr Phe Thr Ser Tyr
20 25 30

Asn Met His Trp Val Lys Gln Thr Pro Arg Gln Gly Leu Glu Trp Ile
35 40 45

Gly Gly Ile Tyr Pro Gly Asn Gly Asp Thr Ser Tyr Asn Gln Lys Phe
50 55 60

Lys Gly Lys Ala Thr Leu Thr Val Gly Lys Ser Ser Ser Thr Ala Tyr
65 70 75 80

Met Gln Leu Ser Ser Leu Thr Ser Glu Asp Ser Ala Val Tyr Phe Cys
85 90 95

Ala Arg Tyr Asp Tyr Asn Tyr Ala Met Asp Tyr Trp Gly Gln Gly Thr
100 105 110

Ser Val Thr Val Ser Ser
115

<210> 9
<211> 47
<212> DNA
<213> Artificial Sequence

<220>
<223> Primer

<400> 9
ctcagtacta gtgccgccac catggatttt caagtgcaga ttttcag 47

<210> 10
<211> 46
<212> DNA
<213> Artificial Sequence

<220>
<223> Primer

<400> 10
tgaagacact tggtgcagcc acagtccggt ttatttccag cctggt 46

<210> 11
<211> 5289
<212> DNA
<213> Artificial Sequence

<220>
<223> Vector

<400> 11
gatctcccga tcccctatgg tgcactctca gtacaatctg ctctgatgcc gcatagttaa 60

gccagtatct gctccctgct tgtgtgttgg aggtcgctga gtagtgcgcg agcaaaattt 120

aagctacaac aaggcaaggc ttgaccgaca attgcatgaa gaatctgctt agggttaggc 180

gttttgcgct gcttcgcgat gtacgggcca gatatacgcg tatctgaggg gactagggtg 240

tgtttaggcg aaaagcgggg cttcggttgt acgcggttag gagtcccctc aggatatagt 300

agtttcgctt ttgcataggg agggggaaat gtagtcttat gcaatactct tgtagtcttg 360

caacatggta acgatgagtt agcaacatgc cttacaagga gagaaaaagc accgtgcatg 420

ccgattggtg gaagtaaggt ggtacgatcg tgccttatta ggaaggcaac agacgggtct 480

gacatggatt ggacgaacca ctgaattccg cattgcagag atattgtatt taagtgccta 540

gctcgataca ataaacgcca tttgaccatt caccacattg gtgtgcacct ccaagcttgg 600

taccgagctc ggatccacta gtaacggccg ccagtgtgct ggaattctgc agatatccat 660

cacactggcg gccgctggct gcaccaagtg tcttcatctt cccgccatct gatgagcagt 720

tgaaatctgg aactgcctct gttgtgtgcc tgctgaataa cttctatccc agagaggcca 780

aagtacagtg gaaggtggat aacgccctcc aatcgggtaa ctcccaggag agtgtcacag 840

agcaggacag caaggacagc acctacagcc tcagcagcac cctgacgctg agcaaagcag 900

actacgagaa acacaaagtc tacgcctgcg aagtcaccca tcagggcctg agctcgcccg 960

tcacaaagag cttcaacagg ggagagtgtt agtctagagc tcgctgatca gcctcgactg 1020

tgccttctag ttgccagcca tctgttgttt gcccctcccc cgtgccttcc ttgaccctgg 1080

aaggtgccac tcccactgtc ctttcctaat aaaatgagga aattgcatcg cattgtctga 1140

gtaggtgtca ttctattctg gggggtgggg tggggcagga cagcaagggg gaggattggg 1200

aagacaatag caggcatgct ggggatgcgg tgggctctat ggcttctgag gcggaaagaa 1260

ccagctgggg ctcgactgtg gaatgtgtgt cagttagggt gtggaaagtc cccaggctcc 1320

ccagcaggca gaagtatgca aagcatgcat ctcaattagt cagcaaccag gtgtggaaag 1380

tccccaggct ccccagcagg cagaagtatg caaagcatgc atctcaatta gtcagcaacc 1440
atagtcccgc ccctaactcc gcccatcccg cccctaactc cgcccagttc cgcccattct 1500
ccgccccatg gctgactaat tttttttatt tatgcagagg ccgaggccgc ctcggcctct 1560
gagctattcc agaagtagtg aggaggcttt tttggaggcc taggcttttg caaaaagctt 1620
gggggggggg acagctcagg gctgcgattt cgcgccaaac ttgacggcaa tcctagcgtg 1680
aaggctggta ggattttatc cccgctgcca tcatggttcg accattgaac tgcatcgtcg 1740
ccgtgtccca agatatgggg attggcaaga acggagacct accctggcct ccgctcagga 1800
acgagttcaa gtacttccaa agaatgacca caacctcttc agtggaaggt aaacagaatc 1860
tggtgattat gggtaggaaa acctggttct ccattcctga gaagaatcga cctttaaagg 1920
acagaattaa tatagttctc agtagagaac tcaaagaacc accacgagga gctcattttc 1980
ttgccaaaag tttggatgat gccttaagac ttattgaaca accggaattg gcaagtaaag 2040
tagacatggt ttggatagtc ggaggcagtt ctgtttacca ggaagccatg aatcaaccag 2100
gccacctcag actctttgtg acaaggatca tgcaggaatt tgaaagtgac acgtttttcc 2160
cagaaattga tttggggaaa tataaacttc tcccagaata cccaggcgtc ctctctgagg 2220
tccaggagga aaaaggcatc aagtataagt ttgaagtcta cgagaagaaa gactaacagg 2280
aagatgcttt caagttctct gctcccctcc taaagctatg catttttata agaccatggg 2340
acttttgctg gctttagatc gatctttgtg aaggaacctt acttctgtgg tgtgacataa 2400
ttggacaaac tacctacaga gatttaaagc tctaaggtaa atataaaatt tttaagtgta 2460
taatgtgtta aactactgat tctaattgtt tgtgtatttt agattccaac ctatggaact 2520
gatgaatggg agcagtggtg gaatgccttt aatgaggaaa acctgttttg ctcagaagaa 2580
atgccatcta gtgatgatga ggctactgct gactctcaac attctactcc tccaaaaaag 2640
aagagaaagg tagaagaccc caaggacttt ccttcagaat tgctaagttt tttgagtcat 2700
gctgtgttta gtaatagaac tcttgcttgc tttgctattt acaccacaaa ggaaaaagct 2760
gcactgctat acaagaaaat tatggaaaaa tattctgtaa cctttataag taggcataac 2820
agttataatc ataacatact gttttttctt actccacaca ggcatagagt gtctgctatt 2880
aataactatg ctcaaaaatt gtgtaccttt agctttttaa tttgtaaagg ggttaataag 2940
gaatatttga tgtatagtgc cttgactaga gatcataatc agccatacca catttgtaga 3000
ggttttactt gctttaaaaa acctcccaca cctccccctg aacctgaaac ataaaatgaa 3060
tgcaattgtt gttgttaact tgtttattgc agcttataat ggttacaaat aaagcaatag 3120
catcacaaat ttcacaaata aagcattttt ttcactgcat tctagttgtg gtttgtccaa 3180
actcatcaat gtatcttatc atgtctggat ccgcgtatgg tgcactctca gtacaatctg 3240
ctctgatgcc gcatagttaa gccagccccg acacccgcca acacccgctg acgcgccctg 3300

acgggcttgt ctgctcccgg catccgctta cagacaagct gtgaccgtct ccgggagctg 3360
catgtgtcag aggttttcac cgtcatcacc gaaacgcgcg agacgaaagg gcctcgtgat 3420
acgcctattt ttataggtta atgtcatgat aataatggtt tcttagacgt caggtggcac 3480
ttttcgggga aatgtgcgcg gaacccctat ttgtttattt ttctaaatac attcaaatat 3540
gtatccgctc atgagacaat aaccctgata aatgcttcaa taatattgaa aaaggaagag 3600
tatgagtatt caacatttcc gtgtcgccct tattcccttt tttgcggcat tttgccttcc 3660
tgtttttgct cacccagaaa cgctggtgaa agtaaaagat gctgaagatc agttgggtgc 3720
acgagtgggt tacatcgaac tggatctcaa cagcggtaag atccttgaga gttttcgccc 3780
cgaagaacgt tttccaatga tgagcacttt taaagttctg ctatgtggcg cggtattatc 3840
ccgtattgac gccgggcaag agcaactcgg tcgccgcata cactattctc agaatgactt 3900
ggttgagtac tcaccagtca cagaaaagca tcttacggat ggcatgacag taagagaatt 3960
atgcagtgct gccataacca tgagtgataa cactgcggcc aacttacttc tgacaacgat 4020
cggaggaccg aaggagctaa ccgctttttt gcacaacatg ggggatcatg taactcgcct 4080
tgatcgttgg gaaccggagc tgaatgaagc cataccaaac gacgagcgtg acaccacgat 4140
gcctgtagca atggcaacaa cgttgcgcaa actattaact ggcgaactac ttactctagc 4200
ttcccggcaa caattaatag actggatgga ggcggataaa gttgcaggac cacttctgcg 4260
ctcggccctt ccggctggct ggtttattgc tgataaatct ggagccggtg agcgtgggtc 4320
tcgcggtatc attgcagcac tggggccaga tggtaagccc tcccgtatcg tagttatcta 4380
cacgacgggg agtcaggcaa ctatggatga acgaaataga cagatcgctg agataggtgc 4440
ctcactgatt aagcattggt aactgtcaga ccaagtttac tcatatatac tttagattga 4500
tttaaaactt catttttaat ttaaaaggat ctaggtgaag atcctttttg ataatctcat 4560
gaccaaaatc ccttaacgtg agttttcgtt ccactgagcg tcagaccccg tagaaaagat 4620
caaaggatct tcttgagatc cttttttct gcgcgtaatc tgctgcttgc aaacaaaaaa 4680
accaccgcta ccagcggtgg tttgtttgcc ggatcaagag ctaccaactc tttttccgaa 4740
ggtaactggc ttcagcagag cgcagatacc aaatactgtc cttctagtgt agccgtagtt 4800
aggccaccac ttcaagaact ctgtagcacc gcctacatac ctcgctctgc taatcctgtt 4860
accagtggct gctgccagtg gcgataagtc gtgtcttacc gggttggact caagacgata 4920
gttaccggat aaggcgcagc ggtcgggctg aacggggggt tcgtgcacac agcccagctt 4980
ggagcgaacg acctacaccg aactgagata cctacagcgt gagctatgag aaagcgccac 5040
gcttcccgaa gggagaaagg cggacaggta tccggtaagc ggcagggtcg gaacaggaga 5100
gcgcacgagg gagcttccag ggggaaacgc ctggtatctt tatagtcctg tcgggtttcg 5160

ccacctctga cttgagcgtc gatttttgtg atgctcgtca ggggggcgga gcctatggaa 5220

aaacgccagc aacgcggcct ttttacggtt cctggccttt tgctggcctt ttgctcacat 5280

ggctcgaca 5289

<210> 12
<211> 6275
<212> DNA
<213> Artificial Sequence

<220>
<223> Vector

<400> 12
tcgaggagac ctgcaaagat ggataaagtt ttaaacagag aggaatcttt gcagctaatg 60

gaccttctag gtcttgaaag gagtgggaat tggctccggt gcccgtcagt gggcagagcg 120

cacatcgccc acagtccccg agaagttgtg gggaggggtc ggcaattgaa ccggtgccta 180

gagaaggtgg cgcggggtaa actgggaaag tgatgtcgtg tactggctcc gcctttttcc 240

cgagggtggg ggagaaccgt atataagtgc agtagtcgcc gtgaacgttc tttttcgcaa 300

cgggtttgcc gccagaacac aggtaagtgc cgtgtgtggt tcccgcgggc ctggcctctt 360

tacgggttat ggcccttgcg tgccttgaat tacttccacc tggctgcagt acgtgattct 420

tgatcccgag cttcgggttg gaagtgggtg ggagagttcg aggccttgcg cttaaggagc 480

cccttcgcct cgtgcttgag ttgaggcctg gcctgggcgc tggggccgcc gcgtgcgaat 540

ctggtggcac cttcgcgcct gtctcgctgc tttcgataag tctctagcca tttaaaattt 600

ttgatgacct gctgcgacgc tttttttctg gcaagatagt cttgtaaatg cgggccaaga 660

tctgcacact ggtatttcgg tttttggggc cgcgggcggc gacggggccc gtgcgtccca 720

gcgcacatgt tcggcgaggc ggggcctgcg agcgcggcca ccgagaatcg gacgggggta 780

gtctcaagct ggccggcctg ctctggtgcc tggcctcgcg ccgccgtgta tcgccccgcc 840

ctgggcggca aggctggccc ggtcggcacc agttgcgtga gcggaaagat ggccgcttcc 900

cggccctgct gcagggagct caaaatggag gacgcggcgc tcgggagagc gggcgggtga 960

gtcacccaca caaaggaaaa gggcctttcc gtcctcagcc gtcgcttcat gtgactccac 1020

ggagtaccgg gcgccgtcca ggcacctcga ttagttctcg agcttttgga gtacgtcgtc 1080

tttaggttgg ggggaggggt tttatgcgat ggagtttccc cacactgagt gggtggagac 1140

tgaagttagg ccagcttggc acttgatgta attctccttg gaatttgccc tttttgagtt 1200

tggatcttgg ttcattctca agcctcagac agtggttcaa agtttttttc ttccatttca 1260

ggtgtcgtga ggaattagct tggtacaaac agcaaagctt aaggtactag tgccgccacc 1320

atggattttc aagtgcagat tttcagcttc ctgctaatca gtgcttcagt cataatgtcc 1380

agaggacaaa ttgttctctc ccagtctcca gcaatcctgt ctgcatctcc aggggagaag 1440

gtcacaatga cttgcagggc cagctcaagt gtaagttaca tgcactggta ccagcagaag 1500
ccaggatcct cccccaaacc ctggatttat gccacatcca acctggcttc tggagtccct 1560
gctcgcttca gtggcagtgg gtctgggacc tcttattctt tcacaatcag cagagtggag 1620
gctgaagatg ctgccactta ttactgccag cagtggactt ttaacccacc cacgttcgga 1680
ggggggacca ggctggaaat aaaccggact gtggctgcac caagtgtctt catcttcccg 1740
ccatctgatg agcagttgaa atctggaact gcctctgttg tgtgcctgct gaataacttc 1800
tatcccagag aggccaaagt acagtggaag gtggataacg ccctccaatc gggtaactcc 1860
caggagagtg tcacagagca ggacagcaag gacagcacct acagcctcag cagcaccctg 1920
acgctgagca aagcagacta cgagaaacac aaagtctacg cctgcgaagt cacccatcag 1980
ggcctgagct cgcccgtcac aaagagcttc aacaggggag agtgttagtc tagagctcgc 2040
tgatcagcct cgactgtgcc ttctagttgc cagccatctg ttgtttgccc ctcccccgtg 2100
ccttccttga ccctggaagg tgccactccc actgtccttt cctaataaaa tgaggaaatt 2160
gcatcgcatt gtctgagtag gtgtcattct attctggggg gtggggtggg gcaggacagc 2220
aagggggagg attgggaaga caatagcagg catgctgggg atgcggtggg ctctatggct 2280
tctgaggcgg aaagaaccag ctggggctcg actgtggaat gtgtgtcagt tagggtgtgg 2340
aaagtcccca ggctccccag caggcagaag tatgcaaagc atgcatctca attagtcagc 2400
aaccaggtgt ggaaagtccc caggctcccc agcaggcaga agtatgcaaa gcatgcatct 2460
caattagtca gcaaccatag tcccgcccct aactccgccc atcccgcccc taactccgcc 2520
cagttccgcc cattctccgc cccatggctg actaattttt tttatttatg cagaggccga 2580
ggccgcctcg gcctctgagc tattccagaa gtagtgagga ggcttttttg gaggcctagg 2640
cttttgcaaa aagctttatc cccgctgcca tcatggttcg accattgaac tgcatcgtcg 2700
ccgtgtccca agatatgggg attggcaaga acggagacct accctggcct ccgctcagga 2760
acgagttcaa gtacttccaa agaatgacca caacctcttc agtggaaggt aaacagaatc 2820
tggtgattat gggtaggaaa acctggttct ccattcctga gaagaatcga cctttaaagg 2880
acagaattaa tatagttctc agtagagaac tcaaagaacc accacgagga gctcattttc 2940
ttgccaaaag tttggatgat gccttaagac ttattgaaca accggaattg gcaagtaaag 3000
tagacatggt ttggatagtc ggaggcagtt ctgtttacca ggaagccatg aatcaaccag 3060
gccacctcag actctttgtg acaaggatca tgcaggaatt tgaaagtgac acgtttttcc 3120
cagaaattga tttggggaaa tataaacttc tcccagaata cccaggcgtc ctctctgagg 3180
tccaggagga aaaaggcatc aagtataagt ttgaagtcta cgagaagaaa gactaacagg 3240
aagatgcttt caagttctct gctcccctcc taaagctatg catttttata agaccatggg 3300

actttttgctg gctttagatc gatctttgtg aaggaacctt acttctgtgg tgtgacataa 3360
ttggacaaac tacctacaga gatttaaagc tctaaggtaa atataaaatt tttaagtgta 3420
taatgtgtta aactactgat tctaattgtt tgtgtatttt agattccaac ctatggaact 3480
gatgaatggg agcagtggtg gaatgccttt aatgaggaaa acctgttttg ctcagaagaa 3540
atgccatcta gtgatgatga ggctactgct gactctcaac attctactcc tccaaaaaag 3600
aagagaaagg tagaagaccc caaggacttt ccttcagaat tgctaagttt tttgagtcat 3660
gctgtgttta gtaatagaac tcttgcttgc tttgctattt acaccacaaa ggaaaaagct 3720
gcactgctat acaagaaaat tatggaaaaa tattctgtaa cctttataag taggcataac 3780
agttataatc ataacatact gtttttttctt actccacaca ggcatagagt gtctgctatt 3840
aataactatg ctcaaaaatt gtgtaccttt agctttttaa tttgtaaagg ggttaataag 3900
gaatatttga tgtatagtgc cttgactaga gatcataatc agccatacca catttgtaga 3960
ggttttactt gctttaaaaa acctcccaca cctccccctg aacctgaaac ataaaatgaa 4020
tgcaattgtt gttgttaact tgtttattgc agcttataat ggttacaaat aaagcaatag 4080
catcacaaat ttcacaaata aagcattttt ttcactgcat tctagttgtg gtttgtccaa 4140
actcatcaat gtatcttatc atgtctggat ccgcgtatgg tgcactctca gtacaatctg 4200
ctctgatgcc gcatagttaa gccagccccg acacccgcca acacccgctg acgcgccctg 4260
acgggcttgt ctgctcccgg catccgctta cagacaagct gtgaccgtct ccgggagctg 4320
catgtgtcag aggttttcac cgtcatcacc gaaacgcgcg agacgaaagg gcctcgtgat 4380
acgcctattt ttataggtta atgtcatgat aataatggtt tcttagacgt caggtggcac 4440
ttttcgggga aatgtgcgcg gaacccctat ttgtttattt ttctaaatac attcaaatat 4500
gtatccgctc atgagacaat aaccctgata aatgcttcaa taatattgaa aaaggaagag 4560
tatgagtatt caacatttcc gtgtcgccct tattcccttt tttgcggcat tttgccttcc 4620
tgtttttgct cacccagaaa cgctggtgaa agtaaaagat gctgaagatc agttgggtgc 4680
acgagtgggt tacatcgaac tggatctcaa cagcggtaag atccttgaga gttttcgccc 4740
cgaagaacgt tttccaatga tgagcacttt taaagttctg ctatgtggcg cggtattatc 4800
ccgtattgac gccgggcaag agcaactcgg tcgccgcata cactattctc agaatgactt 4860
ggttgagtac tcaccagtca cagaaaagca tcttacggat ggcatgacag taagagaatt 4920
atgcagtgct gccataacca tgagtgataa cactgcggcc aacttacttc tgacaacgat 4980
cggaggaccg aaggagctaa ccgctttttt gcacaacatg ggggatcatg taactcgcct 5040
tgatcgttgg gaaccggagc tgaatgaagc cataccaaac gacgagcgtg acaccacgat 5100
gcctgtagca atggcaacaa cgttgcgcaa actattaact ggcgaactac ttactctagc 5160
ttcccggcaa caattaatag actggatgga ggcggataaa gttgcaggac cacttctgcg 5220

ctcggccctt ccggctggct ggtttattgc tgataaatct ggagccggtg agcgtgggtc 5280

tcgcggtatc attgcagcac tggggccaga tggtaagccc tcccgtatcg tagttatcta 5340

cacgacgggg agtcaggcaa ctatggatga acgaaataga cagatcgctg agataggtgc 5400

ctcactgatt aagcattggt aactgtcaga ccaagtttac tcatatatac tttagattga 5460

tttaaaactt catttttaat ttaaaaggat ctaggtgaag atcctttttg ataatctcat 5520

gaccaaaatc ccttaacgtg agttttcgtt ccactgagcg tcagaccccg tagaaaagat 5580

caaaggatct tcttgagatc cttttttct gcgcgtaatc tgctgcttgc aaacaaaaaa 5640

accaccgcta ccagcggtgg tttgtttgcc ggatcaagag ctaccaactc tttttccgaa 5700

ggtaactggc ttcagcagag cgcagatacc aaatactgtc cttctagtgt agccgtagtt 5760

aggccaccac ttcaagaact ctgtagcacc gcctacatac ctcgctctgc taatcctgtt 5820

accagtggct gctgccagtg gcgataagtc gtgtcttacc gggttggact caagacgata 5880

gttaccggat aaggcgcagc ggtcgggctg aacggggggt tcgtgcacac agcccagctt 5940

ggagcgaacg acctacaccg aactgagata cctacagcgt gagctatgag aaagcgccac 6000

gcttcccgaa gggagaaagg cggacaggta tccggtaagc ggcagggtcg gaacaggaga 6060

gcgcacgagg gagcttccag ggggaaacgc ctggtatctt tatagtcctg tcgggtttcg 6120

ccacctctga cttgagcgtc gatttttgtg atgctcgtca ggggggcgga gcctatggaa 6180

aaacgccagc aacgcggcct ttttacggtt cctggccttt tgctggcctt ttgctcacat 6240

ggctcgacag atccgacgga tcgggagatc ctagc 6275

<210> 13
<211> 639
<212> DNA
<213> Artificial Sequence

<220>
<223> Synthetic Construct

<400> 13
caaattgttc tctcccagtc tccagcaatc ctgtctgcat ctccagggga gaaggtcaca 60

atgacttgca gggccagctc aagtgtaagt tacatgcact ggtaccagca gaagccagga 120

tcctccccca aaccctggat ttatgccaca tccaacctgg cttctggagt ccctgctcgc 180

ttcagtggca gtgggtctgg gacctcttat tctttcacaa tcagcagagt ggaggctgaa 240

gatgctgcca cttattactg ccagcagtgg acttttaacc cacccacgtt cggagggggg 300

accaggctgg aaataaaccg gactgtggct gcaccaagtg tcttcatctt cccgccatct 360

gatgagcagt tgaaatctgg aactgcctct gttgtgtgcc tgctgaataa cttctatccc 420

agagaggcca aagtacagtg gaaggtggat aacgccctcc aatcgggtaa ctcccaggag 480

agtgtcacag agcaggacag caaggacagc acctacagcc tcagcagcac cctgacgctg 540

agcaaagcag actacgagaa acacaaagtc tacgcctgcg aagtcaccca tcagggcctg 600

agctcgcccg tcacaaagag cttcaacagg ggagagtgt 639

<210> 14
<211> 213
<212> PRT
<213> Artificial Sequence

<220>
<223> Synthetic Construct

<400> 14

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Lys Pro Gly Ser Ser Pro Lys Pro Trp Ile Tyr
35 40 45

Ala Thr Ser Asn Leu Ala Ser Gly Val Pro Ala Arg Phe Ser Gly Ser
50 55 60

Gly Ser Gly Thr Ser Tyr Ser Phe Thr Ile Ser Arg Val Glu Ala Glu
65 70 75 80

Asp Ala Ala Thr Tyr Tyr Cys Gln Gln Trp Thr Phe Asn Pro Pro Thr
85 90 95

Phe Gly Gly Gly Thr Arg Leu Glu Ile Asn Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 15
<211> 45
<212> DNA
<213> Artificial Sequence

<220>
<223> Primer

<400> 15
ctcagtacta gtgccgccac catgggattc agcaggatct ttctc 45

<210> 16
<211> 48
<212> DNA
<213> Artificial Sequence

<220>
<223> Primer

<400> 16
gaccgatggg cccttggtgg aggctgagga gacggtgact gaggttcc 48

<210> 17
<211> 5739
<212> DNA
<213> Artificial Sequence

<220>
<223> Vector

<400> 17
catggctcga cagatctccc gatcccctat ggtgcactct cagtacaatc tgctctgatg 60

ccgcatagtt aagccagtat ctgctccctg cttgtgtgtt ggaggtcgct gagtagtgcg 120

cgagcaaaat ttaagctaca acaaggcaag gcttgaccga caattgcatg aagaatctgc 180

ttagggttag gcgttttgcg ctgcttcgcg atgtacgggc cagatatacg cgtatctgag 240

gggactaggg tgtgtttagg cgaaaagcgg ggcttcggtt gtacgcggtt aggagtcccc 300

tcaggatata gtagtttcgc ttttgcatag ggagggggaa atgtagtctt atgcaatact 360

cttgtagtct tgcaacatgg taacgatgag ttagcaacat gccttacaag gagagaaaaa 420

gcaccgtgca tgccgattgg tggaagtaag gtggtacgat cgtgccttat taggaaggca 480

acagacgggt ctgacatgga ttggacgaac cactgaattc cgcattgcag agatattgta 540

tttaagtgcc tagctcgata caataaacgc catttgacca ttcaccacat tggtgtgcac 600

ctccaagctt ggtaccgagc tcggatccac tagtaacggc cgccagtgtg ctggaattct 660
gcagatatcc atcacactgg cggccgctcc accaagggcc catcggtctt cccoctggca 720
ccctcctcca agagcacctc tgggggcaca gcggccctgg gctgcctggt caaggactac 780
ttccccgaac cggtgacggt gtcgtggaac tcaggcgccc tgaccagcgg cgtgcacacc 840
ttcccggctg tcctacagtc ctcaggactc tactccctca gcagcgtggt gaccgtgccc 900
tccagcagct tgggcaccca gacctacatc tgcaacgtga atcacaagcc cagcaacacc 960
aaggtggaca agaaagttga gcccaaatct tgtgacaaaa ctcacacatg cccaccgtgc 1020
ccagcacctg aactcctggg gggaccgtca gtcttcctct tccccccaaa acccaaggac 1080
accctcatga tctcccggac ccctgaggtc acatgcgtgg tggtggacgt gagccacgaa 1140
gaccctgagg tcaagttcaa ctggtacgtg gacggcgtgg aggtgcataa tgccaagaca 1200
aagccgcggg aggagcagta caacagcacg taccgtgtgg tcagcgtcct caccgtcctg 1260
caccaggact ggctgaatgg caaggagtac aagtgcaagg tctccaacaa agccctccca 1320
gcccccatcg agaaaaccat ctccaaagcc aaagggcagc cccgagaacc acaggtgtac 1380
accctgcccc catcccggga tgagctgacc aagaaccagg tcagcctgac ctgcctggtc 1440
aaaggcttct atcccagcga catcgccgtg gagtgggaga gcaatgggca gccggagaac 1500
aactacaaga ccacgcctcc cgtgctggac tccgacggct ccttcttcct ctacagcaag 1560
ctcaccgtgg acaagagcag gtggcagcag gggaacgtct tctcatgctc cgtgatgcat 1620
gaggctctgc acaaccacta cacgcagaag agcctctccc tgtctccggg taaatagtct 1680
agagctcgct gatcagcctc gactgtgcct tctagttgcc agccatctgt tgtttgcccc 1740
tcccccgtgc cttccttgac cctggaaggt gccactccca ctgtcctttc ctaataaaat 1800
gaggaaattg catcgcattg tctgagtagg tgtcattcta ttctgggggg tggggtgggg 1860
caggacagca agggggagga ttgggaagac aatagcaggc atgctgggga tgcggtgggc 1920
tctatggctt ctgaggcgga aagaaccagc tggggctcga gcgtgggcca tcgccctgat 1980
agacggtttt tcgccctttg acgttggagt ccacgttctt taatagtgga ctcttgttcc 2040
aaactggaac aacactcaac cctatctcgg tctattcttt tgatttataa gggattttgc 2100
cgatttcggc ctattggtta aaaaatgagc tgatttaaca aatatttaac gcgaatttta 2160
acaaaatatt aacgtttaca atttcgcctg atgcggtatt ttctccttac gcatctgtgc 2220
ggtatttcac accgcatacg cggatctgcg cagcaccatg gcctgaaata acctctgaaa 2280
gaggaacttg gttaggtacc ttctgaggcg gaaagaacca gctgtggaat gtgtgtcagt 2340
tagggtgtgg aaagtcccca ggctccccag caggcagaag tatgcaaagc atgcatctca 2400
attagtcagc aaccaggtgt ggaaagtccc caggctcccc agcaggcaga agtatgcaaa 2460
gcatgcatct caattagtca gcaaccatag tcccgcccct aactccgccc atcccgcccc 2520

taactccgcc cagttccgcc cattctccgc cccatggctg actaattttt tttatttatg 2580

cagaggccga ggccgcctcg gcctctgagc tattccagaa gtagtgagga ggcttttttg 2640

gaggcctagg cttttgcaaa aagcttgatt cttctgacac aacagtctcg aacttaaggc 2700

tagagccacc atgattgaac aagatggatt gcacgcaggt tctccggccg cttgggtgga 2760

gaggctattc ggctatgact gggcacaaca gacaatcggc tgctctgatg ccgccgtgtt 2820

ccggctgtca gcgcaggggc gcccggttct ttttgtcaag accgacctgt ccggtgccct 2880

gaatgaactg caggacgagg cagcgcggct atcgtggctg gccacgacgg gcgttccttg 2940

cgcagctgtg ctcgacgttg tcactgaagc gggaagggac tggctgctat tgggcgaagt 3000

gccggggcag gatctcctgt catctcacct tgctcctgcc gagaaagtat ccatcatggc 3060

tgatgcaatg cggcggctgc atacgcttga tccggctacc tgcccattcg accaccaagc 3120

gaaacatcgc atcgagcgag cacgtactcg gatggaagcc ggtcttgtcg atcaggatga 3180

tctggacgaa gagcatcagg ggctcgcgcc agccgaactg ttcgccaggc tcaaggcgcg 3240

catgcccgac ggcgaggatc tcgtcgtgac ccatggcgat gcctgcttgc cgaatatcat 3300

ggtggaaaat ggccgctttt ctggattcat cgactgtggc cggctgggtg tggcggaccg 3360

ctatcaggac atagcgttgg ctacccgtga tattgctgaa gagcttggcg gcgaatgggc 3420

tgaccgcttc ctcgtgcttt acggtatcgc cgctcccgat tcgcagcgca tcgccttcta 3480

tcgccttctt gacgagttct tctgagcggg actctggggt tcgaaatgac cgaccaagcg 3540

acgcccaacc tgccatcacg atggccgcaa taaaatatct ttattttcat tacatctgtg 3600

tgttggtttt ttgtgtgaat cgatagcgat aaggatcgat cctctagcta gagtcgatcg 3660

acctgcaggg atccgcgtat ggtgcactct cagtacaatc tgctctgatg ccgcatagtt 3720

aagccagccc cgacacccgc caacacccgc tgacgcgccc tgacgggctt gtctgctccc 3780

ggcatccgct tacagacaag ctgtgaccgt ctccgggagc tgcatgtgtc agaggttttc 3840

accgtcatca ccgaaacgcg cgagacgaaa gggcctcgtg atacgcctat ttttataggt 3900

taatgtcatg ataataatgg tttcttagac gtcaggtggc acttttcggg gaaatgtgcg 3960

cggaacccct atttgtttat ttttctaaat acattcaaat atgtatccgc tcatgagaca 4020

ataaccctga taaatgcttc aataatattg aaaaaggaag agtatgagta ttcaacattt 4080

ccgtgtcgcc cttattccct tttttgcggc attttgcctt cctgtttttg ctcacccaga 4140

aacgctggtg aaagtaaaag atgctgaaga tcagttgggt gcacgagtgg gttacatcga 4200

actggatctc aacagcggta agatccttga gagttttcgc cccgaagaac gttttccaat 4260

gatgagcact tttaaagttc tgctatgtgg cgcggtatta tcccgtattg acgccgggca 4320

agagcaactc ggtcgccgca tacactattc tcagaatgac ttggttgagt actcaccagt 4380

cacagaaaag catcttacgg atggcatgac agtaagagaa ttatgcagtg ctgccataac 4440

catgagtgat aacactgcgg ccaacttact tctgacaacg atcggaggac cgaaggagct 4500

aaccgctttt ttgcacaaca tgggggatca tgtaactcgc cttgatcgtt gggaaccgga 4560

gctgaatgaa gccataccaa acgacgagcg tgacaccacg atgcctgtag caatggcaac 4620

aacgttgcgc aaactattaa ctggcgaact acttactcta gcttcccggc aacaattaat 4680

agactggatg gaggcggata aagttgcagg accacttctg cgctcggccc ttccggctgg 4740

ctggtttatt gctgataaat ctggagccgg tgagcgtggg tctcgcggta tcattgcagc 4800

actggggcca gatggtaagc cctcccgtat cgtagttatc tacacgacgg ggagtcaggc 4860

aactatggat gaacgaaata gacagatcgc tgagataggt gcctcactga ttaagcattg 4920

gtaactgtca gaccaagttt actcatatat actttagatt gatttaaaac ttcattttta 4980

atttaaaagg atctaggtga agatcctttt tgataatctc atgaccaaaa tcccttaacg 5040

tgagttttcg ttccactgag cgtcagaccc cgtagaaaag atcaaaggat cttcttgaga 5100

tccttttttt ctgcgcgtaa tctgctgctt gcaaacaaaa aaaccaccgc taccagcggt 5160

ggtttgtttg ccggatcaag agctaccaac tctttttccg aaggtaactg gcttcagcag 5220

agcgcagata ccaaatactg tccttctagt gtagccgtag ttaggccacc acttcaagaa 5280

ctctgtagca ccgcctacat acctcgctct gctaatcctg ttaccagtgg ctgctgccag 5340

tggcgataag tcgtgtctta ccgggttgga ctcaagacga tagttaccgg ataaggcgca 5400

gcggtcgggc tgaacggggg gttcgtgcac acagcccagc ttggagcgaa cgacctacac 5460

cgaactgaga tacctacagc gtgagctatg agaaagcgcc acgcttcccg aagggagaaa 5520

ggcggacagg tatccggtaa gcggcagggt cggaacagga gagcgcacga gggagcttcc 5580

agggggaaac gcctggtatc tttatagtcc tgtcgggttt cgccacctct gacttgagcg 5640

tcgatttttg tgatgctcgt caggggggcg gagcctatgg aaaaacgcca gcaacgcggc 5700

ctttttacgg ttcctggcct tttgctggcc ttttgctca 5739

<210> 18
<211> 6824
<212> DNA
<213> Artificial Sequence

<220>
<223> Vector

<400> 18
tcgaggagac ctgcaaagat ggataaagtt ttaaacagag aggaatcttt gcagctaatg 60

gaccttctag gtcttgaaag gagtgggaat tggctccggt gcccgtcagt gggcagagcg 120

cacatcgccc acagtccccg agaagttgtg gggaggggtc ggcaattgaa ccggtgccta 180

gagaaggtgg cgcggggtaa actgggaaag tgatgtcgtg tactggctcc gcctttttcc 240

cgagggtggg ggagaaccgt atataagtgc agtagtcgcc gtgaacgttc tttttcgcaa 300
cgggtttgcc gccagaacac aggtaagtgc cgtgtgtggt tcccgcgggc ctggcctctt 360
tacgggttat ggcccttgcg tgccttgaat tacttccacc tggctgcagt acgtgattct 420
tgatcccgag cttcgggttg gaagtgggtg ggagagttcg aggccttgcg cttaaggagc 480
cccttcgcct cgtgcttgag ttgaggcctg gcctgggcgc tggggccgcc gcgtgcgaat 540
ctggtggcac cttcgcgcct gtctcgctgc tttcgataag tctctagcca tttaaaattt 600
ttgatgacct gctgcgacgc tttttttctg gcaagatagt cttgtaaatg cgggccaaga 660
tctgcacact ggtatttcgg tttttggggc cgcgggcggc gacggggccc gtgcgtccca 720
gcgcacatgt tcggcgaggc ggggcctgcg agcgcggcca ccgagaatcg gacgggggta 780
gtctcaagct ggccggcctg ctctggtgcc tggcctcgcg ccgccgtgta tcgccccgcc 840
ctgggcggca aggctggccc ggtcggcacc agttgcgtga gcggaaagat ggccgcttcc 900
cggccctgct gcagggagct caaaatggag gacgcggcgc tcgggagagc gggcgggtga 960
gtcacccaca caaaggaaaa gggcctttcc gtcctcagcc gtcgcttcat gtgactccac 1020
ggagtaccgg gcgccgtcca ggcacctcga ttagttctcg agcttttgga gtacgtcgtc 1080
tttaggttgg ggggaggggt tttatgcgat ggagtttccc cacactgagt gggtggagac 1140
tgaagttagg ccagcttggc acttgatgta attctccttg gaatttgccc tttttgagtt 1200
tggatcttgg ttcattctca agcctcagac agtggttcaa agtttttttc ttccatttca 1260
ggtgtcgtga ggaattagct tggtacaaac agcaaagctt aaggtactag tgccgccacc 1320
atgggattca gcaggatctt tctcttcctc ctgtcagtaa ctacaggtgt ccactcccag 1380
gcttatctac agcagtctgg ggctgagctg gtgaggcctg gggcctcagt gaagatgtcc 1440
tgcaaggctt ctggctacac atttaccagt tacaatatgc actgggtaaa gcagacacct 1500
agacagggcc tggaatggat tggaggtatt tatccaggaa atggtgatac ttcctacaat 1560
cagaagttca agggcaaggc cacactgact gtaggcaaat cctccagcac agcctacatg 1620
cagctcagca gcctgacatc tgaagactct gcggtctatt tctgtgcaag atatgactac 1680
aactatgcta tggactactg gggtcaagga acctcagtca ccgtctcctc agcctccacc 1740
aagggcccat cggtcttccc cctggcaccc tcctccaaga gcacctctgg gggcacagcg 1800
gccctgggct gcctggtcaa ggactacttc cccgaaccgg tgacggtgtc gtggaactca 1860
ggcgccctga ccagcggcgt gcacaccttc ccggctgtcc tacagtcctc aggactctac 1920
tccctcagca gcgtggtgac cgtgccctcc agcagcttgg gcacccagac ctacatctgc 1980
aacgtgaatc acaagcccag caacaccaag gtggacaaga aagttgagcc caaatcttgt 2040
gacaaaactc acacatgccc accgtgccca gcacctgaac tcctgggggg accgtcagtc 2100

ttcctcttcc ccccaaaacc caaggacacc ctcatgatct cccggacccc tgaggtcaca 2160

tgcgtggtgg tggacgtgag ccacgaagac cctgaggtca agttcaactg gtacgtggac 2220

ggcgtggagg tgcataatgc caagacaaag ccgcgggagg agcagtacaa cagcacgtac 2280

cgtgtggtca gcgtcctcac cgtcctgcac caggactggc tgaatggcaa ggagtacaag 2340

tgcaaggtct ccaacaaagc cctcccagcc cccatcgaga aaaccatctc caaagccaaa 2400

gggcagcccc gagaaccaca ggtgtacacc ctgcccccat cccgggatga gctgaccaag 2460

aaccaggtca gcctgacctg cctggtcaaa ggcttctatc ccagcgacat cgccgtggag 2520

tgggagagca atgggcagcc ggagaacaac tacaagacca cgcctcccgt gctggactcc 2580

gacggctcct tcttcctcta cagcaagctc accgtggaca agagcaggtg gcagcagggg 2640

aacgtcttct catgctccgt gatgcatgag gctctgcaca accactacac gcagaagagc 2700

ctctccctgt ctccgggtaa atagtctaga gctcgctgat cagcctcgac tgtgccttct 2760

agttgccagc catctgttgt ttgcccctcc cccgtgcctt ccttgaccct ggaaggtgcc 2820

actcccactg tcctttccta ataaaatgag gaaattgcat cgcattgtct gagtaggtgt 2880

cattctattc tggggggtgg ggtggggcag gacagcaagg gggaggattg ggaagacaat 2940

agcaggcatg ctggggatgc ggtgggctct atggcttctg aggcggaaag aaccagctgg 3000

ggctcgagcg tgggccatcg ccctgataga cggtttttcg ccctttgacg ttggagtcca 3060

cgttctttaa tagtggactc ttgttccaaa ctggaacaac actcaaccct atctcggtct 3120

attcttttga tttataaggg attttgccga tttcggccta ttggttaaaa aatgagctga 3180

tttaacaaat atttaacgcg aattttaaca aaatattaac gtttacaatt tcgcctgatg 3240

cggtattttc tccttacgca tctgtgcggt atttcacacc gcatacgcgg atctgcgcag 3300

caccatggcc tgaaataacc tctgaaagag gaacttggtt aggtaccttc tgaggcggaa 3360

agaaccagct gtggaatgtg tgtcagttag ggtgtggaaa gtccccaggc tccccagcag 3420

gcagaagtat gcaaagcatg catctcaatt agtcagcaac caggtgtgga aagtccccag 3480

gctccccagc aggcagaagt atgcaaagca tgcatctcaa ttagtcagca accatagtcc 3540

cgcccctaac tccgcccatc ccgcccctaa ctccgcccag ttccgcccat tctccgcccc 3600

atggctgact aatttttttt atttatgcag aggccgaggc cgcctcggcc tctgagctat 3660

tccagaagta gtgaggaggc ttttttggag gcctaggctt ttgcaaaaag cttgattctt 3720

ctgacacaac agtctcgaac ttaaggctag agccaccatg attgaacaag atggattgca 3780

cgcaggttct ccggccgctt gggtggagag gctattcggc tatgactggg cacaacagac 3840

aatcggctgc tctgatgccg ccgtgttccg gctgtcagcg caggggcgcc cggttctttt 3900

tgtcaagacc gacctgtccg gtgccctgaa tgaactgcag gacgaggcag cgcggctatc 3960

gtggctggcc acgacgggcg ttccttgcgc agctgtgctc gacgttgtca ctgaagcggg 4020

aagggactgg ctgctattgg gcgaagtgcc ggggcaggat ctcctgtcat ctcaccttgc 4080

tcctgccgag aaagtatcca tcatggctga tgcaatgcgg cggctgcata cgcttgatcc 4140

ggctacctgc ccattcgacc accaagcgaa acatcgcatc gagcgagcac gtactcggat 4200

ggaagccggt cttgtcgatc aggatgatct ggacgaagag catcaggggc tcgcgccagc 4260

cgaactgttc gccaggctca aggcgcgcat gcccgacggc gaggatctcg tcgtgaccca 4320

tggcgatgcc tgcttgccga atatcatggt ggaaaatggc cgcttttctg gattcatcga 4380

ctgtggccgg ctgggtgtgg cggaccgcta tcaggacata gcgttggcta cccgtgatat 4440

tgctgaagag cttggcggcg aatgggctga ccgcttcctc gtgctttacg gtatcgccgc 4500

tcccgattcg cagcgcatcg ccttctatcg ccttcttgac gagttcttct gagcgggact 4560

ctggggttcg aaatgaccga ccaagcgacg cccaacctgc catcacgatg gccgcaataa 4620

aatatcttta ttttcattac atctgtgtgt tggttttttg tgtgaatcga tagcgataag 4680

gatcgatcct ctagctagag tcgatcgacc tgcagggatc cgcgtatggt gcactctcag 4740

tacaatctgc tctgatgccg catagttaag ccagccccga cacccgccaa cacccgctga 4800

cgcgccctga cgggcttgtc tgctcccggc atccgcttac agacaagctg tgaccgtctc 4860

cgggagctgc atgtgtcaga ggttttcacc gtcatcaccg aaacgcgcga gacgaaaggg 4920

cctcgtgata cgcctatttt tataggttaa tgtcatgata ataatggttt cttagacgtc 4980

aggtggcact tttcggggaa atgtgcgcgg aacccctatt tgtttatttt tctaaataca 5040

ttcaaatatg tatccgctca tgagacaata accctgataa atgcttcaat aatattgaaa 5100

aaggaagagt atgagtattc aacatttccg tgtcgccctt attccctttt ttgcggcatt 5160

ttgccttcct gtttttgctc acccagaaac gctggtgaaa gtaaaagatg ctgaagatca 5220

gttgggtgca cgagtgggtt acatcgaact ggatctcaac agcggtaaga tccttgagag 5280

ttttcgcccc gaagaacgtt ttccaatgat gagcactttt aaagttctgc tatgtggcgc 5340

ggtattatcc cgtattgacg ccgggcaaga gcaactcggt cgccgcatac actattctca 5400

gaatgacttg gttgagtact caccagtcac agaaaagcat cttacggatg gcatgacagt 5460

aagagaatta tgcagtgctg ccataaccat gagtgataac actgcggcca acttacttct 5520

gacaacgatc ggaggaccga aggagctaac cgcttttttg cacaacatgg gggatcatgt 5580

aactcgcctt gatcgttggg aaccggagct gaatgaagcc ataccaaacg acgagcgtga 5640

caccacgatg cctgtagcaa tggcaacaac gttgcgcaaa ctattaactg gcgaactact 5700

tactctagct tcccggcaac aattaataga ctggatggag gcggataaag ttgcaggacc 5760

acttctgcgc tcggcccttc cggctggctg gtttattgct gataaatctg gagccggtga 5820

gcgtgggtct cgcggtatca ttgcagcact ggggccagat ggtaagccct cccgtatcgt 5880

agttatctac acgacgggga gtcaggcaac tatggatgaa cgaaatagac agatcgctga 5940

gataggtgcc tcactgatta agcattggta actgtcagac caagtttact catatatact 6000

ttagattgat ttaaaacttc atttttaatt taaaaggatc taggtgaaga tcctttttga 6060

taatctcatg accaaaatcc cttaacgtga gttttcgttc cactgagcgt cagaccccgt 6120

agaaaagatc aaaggatctt cttgagatcc tttttttctg cgcgtaatct gctgcttgca 6180

aacaaaaaaa ccaccgctac cagcggtggt ttgtttgccg gatcaagagc taccaactct 6240

ttttccgaag gtaactggct tcagcagagc gcagatacca aatactgtcc ttctagtgta 6300

gccgtagtta ggccaccact tcaagaactc tgtagcaccg cctacatacc tcgctctgct 6360

aatcctgtta ccagtggctg ctgccagtgg cgataagtcg tgtcttaccg ggttggactc 6420

aagacgatag ttaccggata aggcgcagcg gtcgggctga acggggggtt cgtgcacaca 6480

gcccagcttg gagcgaacga cctacaccga actgagatac ctacagcgtg agctatgaga 6540

aagcgccacg cttcccgaag ggagaaaggc ggacaggtat ccggtaagcg gcagggtcgg 6600

aacaggagag cgcacgaggg agcttccagg gggaaacgcc tggtatcttt atagtcctgt 6660

cgggtttcgc cacctctgac ttgagcgtcg atttttgtga tgctcgtcag gggggcggag 6720

cctatggaaa aacgccagca acgcggcctt tttacggttc ctggcctttt gctggccttt 6780

tgctcacatg gctcgacaga tccgacggat cgggagatcc tagc 6824

<210> 19
<211> 1344
<212> DNA
<213> Artificial Sequence

<220>
<223> Synthetic Construct

<400> 19
caggcttatc tacagcagtc tggggctgag ctggtgaggc ctggggcctc agtgaagatg 60

tcctgcaagg cttctggcta cacatttacc agttacaata tgcactgggt aaagcagaca 120

cctagacagg gcctggaatg gattggaggt atttatccag gaaatggtga tacttcctac 180

aatcagaagt tcaagggcaa ggccacactg actgtaggca aatcctccag cacagcctac 240

atgcagctca gcagcctgac atctgaagac tctgcggtct atttctgtgc aagatatgac 300

tacaactatg ctatggacta ctggggtcaa ggaacctcag tcaccgtctc ctcagcctcc 360

accaagggcc catcggtctt ccccctggca ccctcctcca agagcacctc tgggggcaca 420

gcggccctgg gctgcctggt caaggactac ttccccgaac cggtgacggt gtcgtggaac 480

tcaggcgccc tgaccagcgg cgtgcacacc ttcccggctg tcctacagtc ctcaggactc 540

tactccctca gcagcgtggt gaccgtgccc tccagcagct tgggcaccca gacctacatc 600

tgcaacgtga atcacaagcc cagcaacacc aaggtggaca agaaagttga gcccaaatct 660

```
tgtgacaaaa ctcacacatg cccaccgtgc ccagcacctg aactcctggg gggaccgtca    720

gtcttcctct tccccccaaa acccaaggac accctcatga tctcccggac ccctgaggtc    780

acatgcgtgg tggtggacgt gagccacgaa gaccctgagg tcaagttcaa ctggtacgtg    840

gacggcgtgg aggtgcataa tgccaagaca aagccgcggg aggagcagta caacagcacg    900

taccgtgtgg tcagcgtcct caccgtcctg caccaggact ggctgaatgg caaggagtac    960

aagtgcaagg tctccaacaa agccctccca gcccccatcg agaaaaccat ctccaaagcc   1020

aaagggcagc cccgagaacc acaggtgtac accctgcccc catcccggga tgagctgacc   1080

aagaaccagg tcagcctgac ctgcctggtc aaaggcttct atcccagcga catcgccgtg   1140

gagtgggaga gcaatgggca gccggagaac aactacaaga ccacgcctcc cgtgctggac   1200

tccgacggct ccttcttcct ctacagcaag ctcaccgtgg acaagagcag gtggcagcag   1260

gggaacgtct tctcatgctc cgtgatgcat gaggctctgc acaaccacta cacgcagaag   1320

agcctctccc tgtctccggg taaa                                          1344
```

<210> 20
<211> 448
<212> PRT
<213> Artificial Sequence

<220>
<223> Synthetic Construct

<400> 20

```
Gln Ala Tyr Leu Gln Gln Ser Gly Ala Glu Leu Val Arg Pro Gly Ala
1               5                   10                  15

Ser Val Lys Met Ser Cys Lys Ala Ser Gly Tyr Thr Phe Thr Ser Tyr
            20                  25                  30

Asn Met His Trp Val Lys Gln Thr Pro Arg Gln Gly Leu Glu Trp Ile
        35                  40                  45

Gly Gly Ile Tyr Pro Gly Asn Gly Asp Thr Ser Tyr Asn Gln Lys Phe
    50                  55                  60

Lys Gly Lys Ala Thr Leu Thr Val Gly Lys Ser Ser Ser Thr Ala Tyr
65                  70                  75                  80

Met Gln Leu Ser Ser Leu Thr Ser Glu Asp Ser Ala Val Tyr Phe Cys
                85                  90                  95

Ala Arg Tyr Asp Tyr Asn Tyr Ala Met Asp Tyr Trp Gly Gln Gly Thr
            100                 105                 110
```

```
Ser Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Asp Glu Leu Thr Lys Asn Gln Val Ser Leu Thr Cys
        355                 360                 365
```

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210> 21
<211> 318
<212> DNA
<213> Homo sapiens

<400> 21
actgtggctg caccaagtgt cttcatcttc ccgccatctg atgagcagtt gaaatctgga 60

actgcctctg ttgtgtgcct gctgaataac ttctatccca gagaggccaa agtacagtgg 120

aaggtggata acgccctcca atcgggtaac tcccaggaga gtgtcacaga gcaggacagc 180

aaggacagca cctacagcct cagcagcacc ctgacgctga gcaaagcaga ctacgagaaa 240

cacaaagtct acgcctgcga agtcacccat cagggcctga gctcgcccgt cacaaagagc 300

ttcaacaggg gagagtgt 318

<210> 22
<211> 106
<212> PRT
<213> Homo sapiens

<400> 22

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65 70 75 80

His Lys Val Tyr Ala Cys Glu Val Thr His Gln Gly Leu Ser Ser Pro
85 90 95

Val Thr Lys Ser Phe Asn Arg Gly Glu Cys
100 105

<210> 23
<211> 990
<212> DNA
<213> Homo sapiens

<400> 23
gcctccacca agggcccatc ggtcttcccc ctggcaccct cctccaagag cacctctggg 60

ggcacagcgg ccctgggctg cctggtcaag gactacttcc ccgaaccggt gacggtgtcg 120

tggaactcag gcgccctgac cagcggcgtg cacaccttcc cggctgtcct acagtcctca 180

ggactctact ccctcagcag cgtggtgacc gtgccctcca gcagcttggg cacccagacc 240

tacatctgca acgtgaatca caagcccagc aacaccaagg tggacaagaa agttgagccc 300

aaatcttgtg acaaaactca cacatgccca ccgtgcccag cacctgaact cctgggggga 360

ccgtcagtct tcctcttccc cccaaaaccc aaggacaccc tcatgatctc ccggacccct 420

gaggtcacat gcgtggtggt ggacgtgagc cacgaagacc ctgaggtcaa gttcaactgg 480

tacgtggacg gcgtggaggt gcataatgcc aagacaaagc cgcgggagga gcagtacaac 540

agcacgtacc gtgtggtcag cgtcctcacc gtcctgcacc aggactggct gaatggcaag 600

gagtacaagt gcaaggtctc caacaaagcc ctcccagccc ccatcgagaa aaccatctcc 660

aaagccaaag ggcagccccg agaaccacag gtgtacaccc tgcccccatc ccgggatgag 720

ctgaccaaga accaggtcag cctgacctgc ctggtcaaag gcttctatcc cagcgacatc 780

gccgtggagt gggagagcaa tgggcagccg gagaacaact acaagaccac gcctcccgtg 840

ctggactccg acggctcctt cttcctctac agcaagctca ccgtggacaa gagcaggtgg 900

cagcagggga acgtcttctc atgctccgtg atgcatgagg ctctgcacaa ccactacacg 960

cagaagagcc tctccctgtc tccgggtaaa 990

<210> 24
<211> 330
<212> PRT
<213> Homo sapiens

<400> 24

Ala Ser Thr Lys Gly Pro Ser Val Phe Pro Leu Ala Pro Ser Ser Lys
1 5 10 15

```
Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Glu Lys Thr Ile Ser Lys Ala Lys Gly
    210                 215                 220

Gln Pro Arg Glu Pro Gln Val Tyr Thr Leu Pro Pro Ser Arg Asp Glu
225                 230                 235                 240

Leu Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
```

260 265 270

Asn Tyr Lys Thr Thr Pro Pro Val Leu Asp Ser Asp Gly Ser Phe Phe
275 280 285

Leu Tyr Ser Lys Leu Thr Val Asp Lys Ser Arg Trp Gln Gln Gly Asn
290 295 300

Val Phe Ser Cys Ser Val Met His Glu Ala Leu His Asn His Tyr Thr
305 310 315 320

Gln Lys Ser Leu Ser Leu Ser Pro Gly Lys
325 330

<210> 25
<211> 321
<212> DNA
<213> Mus musculus

<400> 25
caaattgttc tctcccagtc tccagcaatc ctgtctgcat ctccagggga gaaggtcaca 60

atgacttgca gggccagctc aagtgtaagt tacatgcact ggtaccagca gaagccagga 120

tcctccccca aaccctggat ttatgccaca tccaacctgg cttctggagt ccctgctcgc 180

ttcagtggca gtgggtctgg gacctcttat tctttcacaa tcagcagagt ggaggctgaa 240

gatgctgcca cttattactg ccagcagtgg acttttaacc cacccacgtt cggagggggg 300

accaggctgg aaataaaacg g 321

<210> 26
<211> 107
<212> PRT
<213> Mus musculus

<400> 26

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Lys Pro Gly Ser Ser Pro Lys Pro Trp Ile Tyr
35 40 45

Ala Thr Ser Asn Leu Ala Ser Gly Val Pro Ala Arg Phe Ser Gly Ser
50 55 60

Gly Ser Gly Thr Ser Tyr Ser Phe Thr Ile Ser Arg Val Glu Ala Glu
65 70 75 80

Asp Ala Ala Thr Tyr Tyr Cys Gln Gln Trp Thr Phe Asn Pro Pro Thr
85 90 95

Phe Gly Gly Gly Thr Arg Leu Glu Ile Lys Arg
100 105

<210> 27
<211> 645
<212> DNA
<213> Artificial Sequence

<220>
<223> Synthetic Construct

<400> 27
caaattgttc tctcccagtc tccagcaatc ctgtctgcat ctccagggga gaaggtcaca 60

atgacttgca gggccagctc aagtgtaagt tacatgcact ggtaccagca gaagccagga 120

tcctcccccca aaccctggat ttatgccaca tccaacctgg cttctggagt ccctgctcgc 180

ttcagtggca gtgggtctgg gacctcttat tctttcacaa tcagcagagt ggaggctgaa 240

gatgctgcca cttattactg ccagcagtgg acttttaacc cacccacgtt cggagggggg 300

accaggctgg aaataaaacg gactgtggct gcaccaagtg tcttcatctt cccgccatct 360

gatgagcagt tgaaatctgg aactgcctct gttgtgtgcc tgctgaataa cttctatccc 420

agagaggcca aagtacagtg gaaggtggat aacgccctcc aatcgggtaa ctcccaggag 480

agtgtcacag agcaggacag caaggacagc acctacagcc tcagcagcac cctgacgctg 540

agcaaagcag actacgagaa acacaaagtc tacgcctgcg aagtcaccca tcagggcctg 600

agctcgcccg tcacaaagag cttcaacagg ggagagtgtt agtga 645

<210> 28
<211> 213
<212> PRT
<213> Artificial Sequence

<220>
<223> Synthetic Construct

<400> 28

Gln Ile Val Leu Ser Gln Ser Pro Ala Ile Leu Ser Ala Ser Pro Gly
1 5 10 15

Glu Lys Val Thr Met Thr Cys Arg Ala Ser Ser Ser Val Ser Tyr Met
20 25 30

His Trp Tyr Gln Gln Lys Pro Gly Ser Ser Pro Lys Pro Trp Ile Tyr
35 40 45

```
Ala Thr Ser Asn Leu Ala Ser Gly Val Pro Ala Arg Phe Ser Gly Ser
    50                  55                  60

Gly Ser Gly Thr Ser Tyr Ser Phe Thr Ile Ser Arg Val Glu Ala Glu
65                  70                  75                  80

Asp Ala Ala Thr Tyr Tyr Cys Gln Gln Trp Thr Phe Asn Pro Pro Thr
                85                  90                  95

Phe Gly Gly Gly Thr Arg Leu Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210


<210>  29
<211>  46
<212>  DNA
<213>  Artificial Sequence

<220>
<223>  Primer

<400>  29
tgaagacact tggtgcagcc acagtccgtt ttatttccag cctggt                    46
```